(19) Europäisches Patentamt
European Patent Office
Office européen des brevets

(11) EP 1 574 205 A2

(12) **DEMANDE DE BREVET EUROPEEN**

(43) Date de publication:
**14.09.2005 Bulletin 2005/37**

(51) Int Cl.[7]: **A61K 7/13**

(21) Numéro de dépôt: **05290441.4**

(22) Date de dépôt: **25.02.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **27.02.2004 FR 0450380**
**25.06.2004 FR 0407022**

(71) Demandeur: **L'OREAL**
**75008 Paris (FR)**

(72) Inventeurs:
- **Greaves, Andrew**
**77144 Montevrain (FR)**
- **David, Hervé**
**94340 Joinville Le Pont (FR)**
- **Samain, Henri**
**91570 Bievres (FR)**

(74) Mandataire: **Wattremez, Catherine**
**L'OREAL - D.I.P.I.**
**25-29 Quai Aulagnier**
**92600 Asnières (FR)**

(54) **Composition comprenant un colorant mixte à base d'au moins un chromophore de type azo ou tri-(hetero)aryle methane**

(57) La présente invention a pour objet une composition tinctoriale comprenant un ou plusieurs colorants mixtes comprenant au moins un chromophore de type azo ou de type tri-(hétéro)aryle méthane.

Elle concerne de plus un procédé de coloration de fibres kératiniques, notamment humaines, mettant en jeu ladite composition, ainsi qu'un dispositif approprié.

Elle a de même pour objet des colorants mixtes en tant que tels.

EP 1 574 205 A2

Printed by Jouve, 75001 PARIS (FR)

## Description

La présente invention a pour objet une composition tinctoriale comprenant un ou plusieurs colorants mixtes comprenant au moins un chromophore de type azo ou de type tri-(hétéro)aryle méthane, ainsi qu'un procédé de coloration de fibres kératiniques, notamment humaines, mettant en jeu ladite composition. Elle a de même pour objet des colorants mixtes en tant que tels.

Il est connu de teindre les fibres kératiniques et en particulier les cheveux humains avec des compositions tinctoriales contenant des colorants directs. Ces composés sont des molécules colorées et colorantes ayant une affinité pour les fibres. Il est connu par exemple d'utiliser des colorants directs du type nitrés benzéniques, des colorants anthraquinoniques, des nitropyridines, des colorants du type azoïque, xanthénique, acridinique azinique ou triarylméthane.

Habituellement, ces colorants sont appliqués sur les fibres, éventuellement en présence d'un agent oxydant, si l'on souhaite obtenir un effet simultané d'éclaircissement des fibres. Une fois le temps de pose écoulé, les fibres sont rincées, éventuellement lavées et séchées.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur relative mauvaise tenue aux lavages ou à la transpiration.

On est aussi confronté à une difficulté supplémentaire, liée au fait que pour obtenir une couleur particulière, il est nécessaire dans la majeure partie des cas, pour ne pas dire la totalité, de mélanger plusieurs colorants. Or chaque colorant ne possède pas la même affinité pour la fibre, ce qui se traduit soit par des colorations hétérogènes, soit par des virages de couleur au cours du temps, par exemple après un ou plusieurs lavages des fibres, exposition au soleil, etc.

Le but de la présente invention est de fournir des colorants directs ne présentant pas les inconvénients des colorants directs existants.

En particulier, l'un des buts de la présente invention est de fournir des colorants directs qui permettent d'obtenir des nuances variées sans problème de virage de la couleur dans le temps.

Ces buts et d'autres sont atteints par la présente invention qui a pour objet une composition tinctoriale comprenant, dans un milieu approprié pour la teinture des fibres kératiniques, notamment humaines, au moins un colorant mixte comprenant au moins deux chromophores différents ; au moins l'un des chromophores étant choisi parmi les chromophores de la famille des composés azo ou parmi les chromophores de la famille des tri-(hétéro)aryle méthanes ; les chromophores étant liés entre eux par l'intermédiaire d'au moins un bras de liaison stoppant la délocalisation des électrons de chacun des chromophores ; les colorants mixtes dont les chromophores sont tous de type azo ne faisant pas partie de la présente invention.

Elle a de même pour objet un procédé de coloration de fibres kératiniques, notamment humaines, dans lequel on applique la composition tinctoriale précitée, éventuellement en présence d'au moins un agent oxydant, on laisse poser pendant une durée suffisante pour obtenir la coloration souhaitée, on rince éventuellement les fibres, on les lave éventuellement et on les rince, puis on les sèche ou on les laisse sécher.

Elle a de plus pour objet des colorants mixtes comprenant au moins deux chromophores différents ; au moins l'un des chromophores étant choisi parmi les chromophores de la famille des composés azo ou parmi les chromophores de la famille des tri-(hétéro)aryle méthanes ; les chromophores étant liés entre eux par l'intermédiaire d'au moins un bras de liaison stoppant la délocalisation des électrons de chacun des chromophores ; les colorants mixtes dont les chromophores sont tous de type azo ne faisant pas partie de la présente invention, et à l'exception des composés suivants :

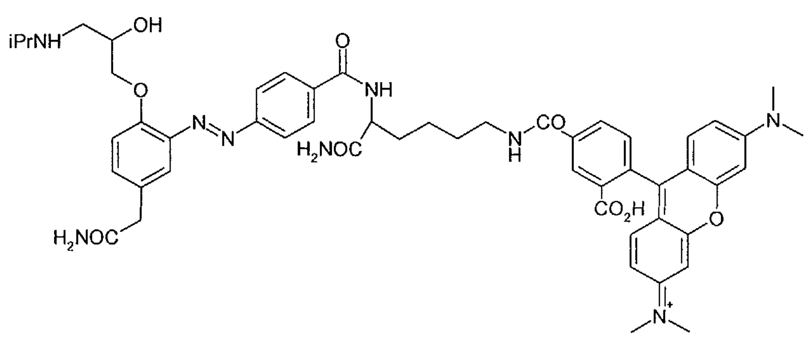

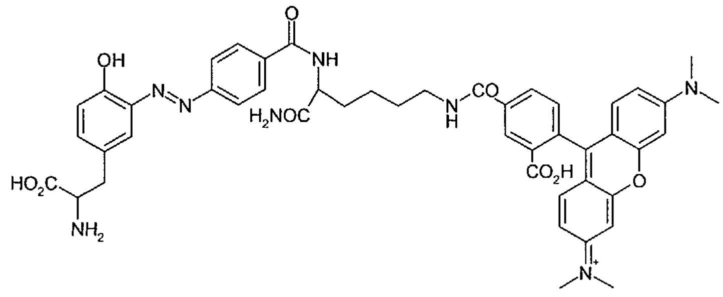

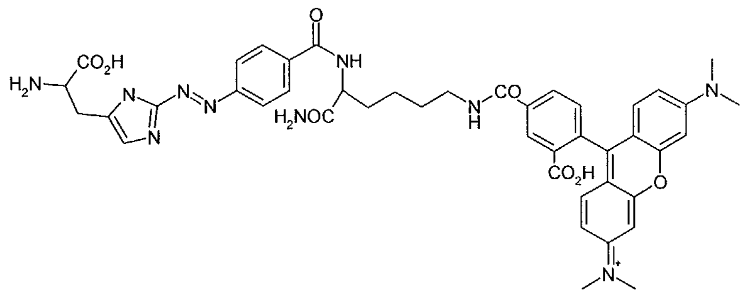

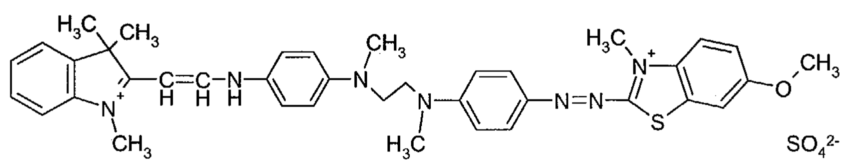

On a constaté que la composition tinctoriale selon l'invention permettait d'obtenir des couleurs puissantes, stables à la lumière, résistantes aux intempéries, aux lavages et à la transpiration, et tenaces dans le temps.

Mais d'autres caractéristiques et avantages de la présente invention apparaîtront plus clairement à la lecture de la description et des exemples qui vont être présentés.

Dans la suite, et à moins d'une indication différente, on entend par radical alkyle substitué, aryle (ou aromatique) substitué ou hétéroaryle (ou hétéroaromatique) substitué, un radical alkyle, aryle ou hétéroaryle portant un ou plusieurs radicaux choisis parmi un radical hydroxyle ; un atome d'halogène, et de préférence le chlore, le fluor ; un radical alcoxy en $C_1$-$C_8$ linéaire ou ramifié, substitué ou non, de préférence en $C_1$-$C_4$ ; un radical monohydroxy alcoxy dont la partie alkyle est en $C_1$-$C_8$, de préférence en $C_1$-$C_4$, linéaire ou ramifiée, substituée ou non ; un radical polyhydroxyalcoxy, en $C_2$-$C_8$, de préférence en $C_2$-$C_4$, linéaire ou ramifié, substitué ou non ; un radical amino substitué ou non par un ou plusieurs radicaux alkyle, identiques ou différents, en $C_1$-$C_8$, de préférence en $C_1$-$C_6$, linéaires ou ramifiés, substitués ou non et/ou par un ou plusieurs radicaux aryles, de préférence en $C_6$, éventuellement substitués ; un radical thiol ; un radical alkylthio en $C_1$-$C_8$, de préférence en $C_1$-$C_4$, linéaire ou ramifiée, substitué ou non ; un radical carboxylique sous forme acide ou salifiée (de préférence avec un métal alcalin ou un ammonium, substitué ou non) ; un radical alcoxycarbonyle dont la partie alkyle est en $C_1$-$C_8$, de préférence en $C_1$-$C_4$, linéaire ou ramifiée, substituée ou non ; un radical alkylamide dont la partie alkyle est en $C_1$-$C_8$, de préférence en $C_1$-$C_4$, linéaire ou ramifié, substitué ou non ; un radical alkylcarbamyle dont la partie alkyle est en $C_1$-$C_8$, de préférence en $C_1$-$C_4$, linéaire ou ramifiée, substituée ou non ; un radical nitro ; un radical sulfonyle ; un radical alkylsulfonyle en $C_1$-$C_8$, de préférence en $C_1$-$C_4$, linéaire ou ramifié substitué ou non ; un radical sulfonylamino ; un radical alkylsulfonylamido dont la partie alkyle est en $C_1$-$C_8$, de préférence en $C_1$-$C_4$, linéaire ou ramifiée, substituée ou non.

Rappelons qu'un radical hétéroaromatique, ou hétéroaryle, correspond à un radical aromatique dans lequel au moins l'un des atomes de carbone est remplacé par un hétéroatome, et plus particulièrement, l'azote, l'oxygène ou le soufre.

De plus, lorsqu'il est indiqué que le radical alkyle ou aryle ou la partie alkyle ou aryle d'un radical substituant un autre radical, est lui-même substitué, on entend qu'il comprend un ou plusieurs substituants choisis parmi les groupements hydroxyle ; amino ; amino substitués par un ou plusieurs radicaux alkyle, identiques ou non, linéaires ou ramifiés, en $C_1$-$C_4$, éventuellement porteurs d'au moins un groupement hydroxyle ; des radicaux alcoxy, linéaires ou

ramifiés en $C_1$-$C_4$, éventuellement porteurs d'au moins un ou plusieurs groupements hydroxyle.

Lorsqu'il est fait mention de radicaux amino portant deux substituants choisis parmi des radicaux alkyle éventuellement substitué, il est entendu que lesdits radicaux alkyle peuvent aussi former avec l'atome d'azote auquel ils sont liés, un cycle à 5 ou 6 chaînons dont l'un au moins des atomes de carbone peut être remplacé par un ou plusieurs atomes d'azote, d'oxygène ou de soufre.

De plus, sauf indication contraire, les bornes délimitant l'étendue d'une plage de valeurs sont comprises dans cette plage de valeurs.

En outre, les colorants mixtes selon l'invention étant cationiques, leur(s) contre-ion(s) sont choisis parmi les anions cosmétiquement acceptables, de nature minérale ou organique. A titre d'exemples d'anions de nature minérale, on peut citer notamment les halogénures, comme les chlorures, les bromures ; les hydroxydes ; les sulfates ; les hydrogénosulfates ; les carbonates, les hydrogénocarbonates.

A titre d'exemples d'anions de nature organique, conviennent les anions tels que l'acétate ; le citrate ; le tartrate ; les alkylsulfates pour lesquels la partie alkyle, linéaire ou ramifiée, est en $C_1$-$C_6$, comme l'ion méthosulfate, éthosulfate ; les alkylsulfonates pour lesquels la partie alkyle, linéaire ou ramifiée, est en $C_1$-$C_6$ ; les arylsulfonates pour lesquels la partie aryle, de préférence phényle, est éventuellement substituée par un ou plusieurs radicaux alkyle en $C_1$-$C_4$.

Les colorants mixtes présents dans la composition selon l'invention vont tout d'abord être décrits.

Comme indiqué précédemment, les colorants mixtes comprennent au moins deux chromophores différents ; au moins l'un des chromophores est choisi parmi les chromophores de la famille des composés azo ou parmi les chromophores de la famille des tri-(hétéro)aryle méthanes ; les chromophores étant liés entre eux par l'intermédiaire d'au moins un bras de liaison stoppant la délocalisation des électrons de chacun des chromophores. Enfin, les colorants mixtes dont les chromophores sont tous de type azo ne font pas partie de la présente invention.

De préférence l'un au moins des chromophores du colorant mixte porte une ou plusieurs charges cationiques.

Par chromophore, on entend un radical issu d'un colorant, c'est-à-dire un radical d'une molécule absorbant dans le domaine visible entre 400 et 800 nm. Il est à noter de plus que cette absorbance du colorant ne nécessite ni oxydation préalable de celui-ci, ni association avec d'autre(s) espèce(s) chimique(s).

Lorsqu'il est précisé que les chromophores sont différents, cela signifie qu'au moins deux d'entre eux, de préférence tous, diffèrent par leur structure chimique. De tels chromophores peuvent être des chromophores issus de familles différentes ou d'une même famille à la condition de présenter des structures chimiques différentes. Par exemple, les chromophores peuvent être choisis dans une même famille de colorants mais différer par la structure chimique des radicaux les constituant.

Selon un mode de réalisation particulier de l'invention, le colorant mixte comprend deux à quatre chromophores, avantageusement deux à trois chromophores.

Il est à noter que lorsque le colorant comprend plus de deux chromophores, au moins l'un de ces chromophores est différent de l'autre ou des autres.

Conformément à un mode de réalisation préféré de l'invention, le colorant mixte comprend deux chromophores.

Conformément à une variante particulière de l'invention, le ou les chromophores cationiques sont des chromophores comprenant au moins un atome d'azote quaternisé.

De plus, la ou les charges cationiques peuvent être engagées ou non dans un cycle.

Par ailleurs, comme indiqué plus haut, au moins l'un des chromophores du colorant mixte présente au moins une charge cationique, et plus particulièrement une seule charge cationique.

De préférence, selon cette variante, chacun des chromophores comprend au moins une charge cationique, et de manière très avantageuse, une seule charge cationique.

Conformément à un mode de réalisation particulier de l'invention, le colorant mixte possède une charge globale cationique, dans les conditions d'utilisation de ce colorant mixte.

Le colorant mixte présent dans la composition selon l'invention comprend donc au moins un chromophore de la famille des composés azo ou au moins un chromophore de la famille des tri-(hétéro)aryle méthanes.

Les chromophores de la famille des composés azo (ou azoïques) sont choisis parmi les composés comprenant au moins un enchaînement -N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Selon une variante, le colorant mixte comprend au moins un chromophore azoïque cationique.

De tels chromophores sont décrits par exemple dans EP 850 636, FR 2788433, EP 920856, WO 9948465, FR 2757385 , EP 850637 , EP 918053, WO 9744004, FR 2570946, FR 2285851, DE 2538363, FR 2189006, FR 1560664 , FR 1540423, FR 1567219, FR 1516943, FR 1221122, DE 4220388, DE 4137005, WO 0166646, US 5708151, WO 9501772, WO 9515144, GB 1195386, US 3524842, US 5879413, EP 1062940, EP 1133976, GB 738585, DE 2527638, FR 2275462, GB 1974-27645, Acta Histochem. (1978), 61(1), 48-52 ; Tsitologiya (1968), 10(3), 403-5 ; Zh. Obshch. Khim. (1970), 40(1), 195-202; Ann. Chim. (Rome) (1975), 65(5-6), 305-14; Journal of the Chinese Chemical Society (Taipei) (1998), 45(1), 209-211 ; Rev. Roum. Chim. (1988), 33(4), 377-83 ; Text. Res. J. (1984), 54(2), 105-7 ; Chim. Ind. (Milan) (1974), 56(9), 600-3 ; Khim. Tekhnol. (1979), 22(5), 548-53 ; Ger. Monatsh. Chem. (1975), 106(3), 643-8 ; MRL Bull. Res. Dev. (1992), 6(2), 21-7 ; Lihua Jianyan, Huaxue Fence (1993), 29(4), 233-4 ; Ann. Chim. (Rome) (1975), 65(5-6), 305-14 ; Dyes Pigm. (1992), 19(1), 69-79 ; Dyes Pigm. (1989), 11(3), 163-72.

A titre d'exemples particulièrement avantageux, le ou les chromophores de la famille des composés azos correspondent à la formule suivante (I), ainsi que ses formes tautomères :

$$A_1 - [N{=}N - (A_3)_y]_x - A_2 \qquad (I)$$

Formule dans laquelle :

- x est un nombre entier compris entre 1 et 3 ;
- y vaut 0 ou 1 ;

De préférence si y est égal à zéro, alors x est égal à 1.

- $A_1$, $A_2$, identiques ou non, représentent un radical aromatique en $C_6$-$C_{30}$ ou un radical hétéroaromatique de 5 à 30 chaînons, comprenant au moins un hétéroatome choisi parmi l'azote, l'oxygène et/ou le soufre ; l'un au moins des groupements $A_1$, $A_2$ étant substitué ;
- $A_1$ ou $A_2$ étant lié au bras de liaison du colorant mixte ;
- $A_3$ représente un radical divalent mono- ou poly-aromatique en $C_6$-$C_{30}$, éventuellement substitué, de préférence par un ou plusieurs groupements, identiques ou non, choisis parmi un radical alkyle, linéaire ou ramifié, en $C_1$-$C_6$ ; alcoxy, linéaire ou ramifié, en $C_1$-$C_6$ ; hydroxyle ; amino ; amino substitué par un ou plusieurs radicaux alkyles, identiques ou non, linéaires ou ramifiés, en $C_1$-$C_6$, éventuellement porteurs d'au moins un radical hydroxyle ; nitro ; par un ou plusieurs atomes d'halogène, alkylsulfonamido en $C_1$-$C_{12}$ (alkyl-$SO_2$-NH-), alkylsulfamoyle en $C_1$-$C_{12}$ (alkyl-NH-$SO_2$-), acyloxy dont la partie alkyle est en en $C_1$-$C_{12}$ ; alcoxycarbonyle dont la partie alkyle est en en $C_1$-$C_{12}$ ; carboxyle.

De préférence, $A_1$, $A_2$, identiques ou non, représentent un radical aromatique en $C_6$-$C_{30}$ ou un radical hétéroaromatique comprenant de 5 à 30 chaînons, comprenant au moins un hétéroatome choisi parmi l'azote, l'oxygène et/ou le soufre ; lesdits radicaux étant éventuellement substitués par au moins un radical alkyle linéaire ou ramifié, en $C_1$-$C_8$, substitué ou non ; au moins un radical (hétéro)aromatique en $C_5$ ou $C_6$, substitué ou non par un ou plusieurs radicaux alcoxy, linéaire ou ramifiés, en $C_1$-$C_6$ ; amino ; amino substitué par un ou plusieurs radicaux alkyles, identiques ou non, linéaires ou ramifiés en $C_1$-$C_6$, éventuellement porteurs d'au moins un groupement hydroxyle ; amido ; hydroxyle ; atome d'halogène ; nitro ; cyano.

De préférence, au moins l'un des groupements $A_1$ ou $A_2$ est cationique.

Selon un mode de réalisation particulièrement avantageux, le ou les chromophores de la famille des composés azos correspondent à la formule (I) dans laquelle x vaut 1 et y vaut 0.

Conformément à un mode de réalisation encore plus particulier de l'invention, le ou les chromophores de la famille des composés azos correspondent à la formule (I) avec x=1 et y=0, ainsi que ses formes tautomères, dans laquelle :

$A_1$ représente

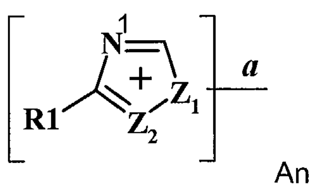

Formule (II)

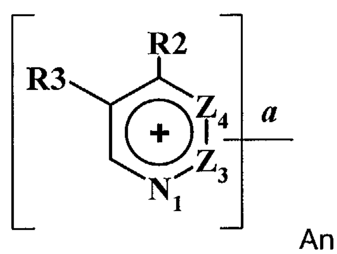

Formule (III)

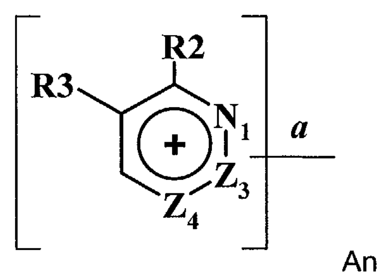

Formule (IV)

la liaison a étant reliée au groupement azoïque soit par l'intermédiaire du cycle ou par l'intermédiaire de $N^1$, $Z_1$, $Z_2$, $Z_3$, $Z_4$, $R_1$, $R_2$, $R_3$ ;
$Z_1$ représente un atome d'oxygène, de soufre, un radical $NR_4$ ou un radical $CR_5$,
$Z_2$ représente un atome d'azote ou un radical $CR_6$,
$Z_3$ représente un atome d'azote ou un radical $CR_7$,
$Z_4$ représente un atome d'azote ou un radical $CR_8$,
$R_1$ et $R_6$ peuvent former ensemble un cycle aromatique,
An représente un anion cosmétiquement acceptable,

$A_2$ représente un groupement aromatique en $C_6$-$C_{30}$ ou hétéroaromatique comprenant de 5 à 30 chaînons, éventuellement substitué, éventuellement cationique, lié au groupement azoïque soit par l'intermédiaire dudit cycle, soit par l'intermédiaire de l'un de ses substituants.

De préférence, $A_2$ représente un groupement aromatique carboné ou pyridinique de formule :

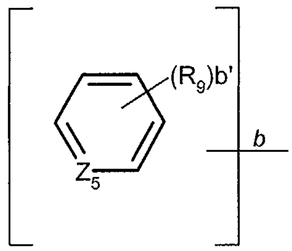

Formule (V)

Formules dans lesquelles :

La liaison b étant reliée au groupement azoïque par l'intermédiaire du cycle ;
$Z_5$ représente un atome d'azote ou un radical $CR_{10}$ ;
$R_1$, $R_2$, $R_3$, $R_5$, $R_6$, $R_7$, $R_8$, $R_9$, et $R_{10}$, identiques ou différents, représentent un atome d'hydrogène ; un radical alkyle, linéaire ou ramifié, en $C_1$-$C_8$, éventuellement substitué ; un radical (hétéro)aryle comprenant 5 ou 6 chaînons, éventuellement substitué ; un atome d'halogène ; un groupement hydroxyle ; un radical alcoxy linéaire ou ramifié, en $C_1$-$C_8$, éventuellement substitué ; un radical amino ; un radical amino substitué par un ou plusieurs radicaux alkyles, identiques ou non, linéaires ou ramifiés en $C_1$-$C_6$, éventuellement porteurs d'au moins un groupement hydroxyle ; un groupement nitro ; un groupement cyano ; alkylsulfonamido en $C_1$-$C_{12}$ (alkyl-$SO_2$-NH-) ; alkylsulfamoyle en $C_1$-$C_{12}$ (alkyl-NH-$SO_2$-) ; acyloxy dont la partie alkyle est en en $C_1$-$C_{12}$ ; alcoxycarbonyle dont la partie alkyle est en en $C_1$-$C_{12}$ ; un groupement carboxy ;
$R_4$ représente un radical alkyle, linéaire ou ramifié, en $C_1$-$C_8$, éventuellement substitué ;
$R_1$ avec $R_5$, et $R_6$ avec $R_7$ peuvent former un cycle aromatique ;
Le coefficient b' est égal à 4 ;
An représente un anion cosmétiquement acceptable de nature organique ou minérale.

De préférence $R_1$, $R_2$, $R_3$, $R_5$, $R_6$, $R_7$, $R_8$, $R_9$ et $R_{10}$, identiques ou non, représentent un atome d'hydrogène, un radical alkyle en $C_1$-$C_4$, linéaire ou ramifié, éventuellement substitué par au moins un atome d'halogène, un groupement nitro, un groupement cyano, un groupement amido (-$CONH_2$), un groupement alcoxy en $C_1$-$C_4$ ; un groupement hydroxyle ; un radical alcoxy linéaire ou ramifié, en $C_1$-$C_8$ ; un radical amino ; un radical amino substitué par un ou plusieurs radicaux alkyles, identiques ou non, linéaires ou ramifiés, en $C_1$-$C_4$, éventuellement porteurs d'un groupement hydroxyle.

De préférence $R_4$ représente un radical alkyle, linéaire ou ramifié, en $C_1$-$C_4$, éventuellement substitué.

Conformément à un premier mode de réalisation avantageux de l'invention, $Z_1$ représente un radical $NR_4$. Par ailleurs, selon ce mode de réalisation, $Z_2$ représente plus préférentiellement $CR_6$.

Conformément à un autre mode de réalisation avantageux de l'invention, $Z_1$ représente un groupement $NR_4$ et $Z_2$ représente un groupement $CR_6$.

Selon ces deux modes de réalisation de l'invention, le groupement azoïque est lié au groupement de formule (II) par l'intermédiaire du cycle.

Selon une autre variante avantageuse de l'invention, le groupement $A_1$ correspond à l'une des formules (III) ou (IV) et le groupement azoïque est lié par l'intermédiaire du cycle ou de $Z_3$ avec $Z_3$ représentant de préférence un

groupement $CR_7$.

En outre, le chromophore est relié au bras de liaison par l'intermédiaire du groupe $A_1$ ou du groupe $A_2$.

Que le chromophore et le bras de liaison soient liés par le groupe $A_1$ ou par le groupe $A_2$, la liaison peut être réalisée soit par l'intermédiaire de l'un des maillons des cycles des groupes de formules (II) à (IV) ou du groupe (hétéro) aromatique $A_2$, et de préférence du groupe de formule (V), soit par l'intermédiaire de l'un des substituants portés par les maillons des cycles précités.

Plus préférentiellement, si le chromophore et le bras de liaison sont reliés par le groupe A1, la liaison peut être obtenue avantageusement par l'intermédiaire de l'atome d'azote $N^1$, les radicaux $R_1$, $R_2$, $R_3$, $R_4$, $R_5$, $R_6$, $R_7$ et $R_8$. Avantageusement, la liaison a lieu par l'intermédiaire de l'atome d'azote $N^1$.

Au cas où le chromophore et le bras de liaison ne sont pas reliés par l'intermédiaire de l'atome d'azote $N^1$, alors l'atome d'azote $N^1$ porte éventuellement un radical, identique ou différent de l'un des radicaux $R_1$ à $R_{10}$, et de préférence par un radical alkyle, linéaire ou ramifié, en $C_1$-$C_4$, éventuellement substitué.

Selon un premier mode de réalisation de l'invention, lorsque le chromophore et le bras de liaison sont liés par le groupe $A_2$, et plus spécialement par un groupe $A_2$ de formule (V), alors le chromophore et le bras de liaison peuvent être liés par l'intermédiaire de l'un des radicaux $R_9$, $R_{10}$ ou par le cycle, avec de préférence dans ce dernier cas, la présence d'un atome d'oxygène ou d'azote directement lié au cycle aromatique. Conformément à une variante préférée de l'invention, le chromophore et le bras de liaison sont liés par l'intermédiaire d'un radical $R_9$ Plus particulièrement, $R_9$ est lié au cycle par l'intermédiaire d'un atome d'azote ou d'oxygène.

Selon un deuxième mode de réalisation de l'invention, le chromophore et le bras de liaison sont reliés par l'intermédiaire du groupe $A_1$ et plus précisément via l'atome d'azote $N^1$.

Selon un troisième mode de réalisation de l'invention, le chromophore et le bras de liaison sont reliés par l'intermédiaire du groupe $A_1$ et plus précisément via le groupe $Z_1$, avantageusement représentant C-$R_5$, de préférence directement par cet atome de carbone. Selon ce mode de réalisation, $R_1$ et $R_6$ peuvent former ensemble un cycle aromatique à 6 chaînons.

Dans le cas où le chromophore est relié au bras de liaison par le groupe $A_2$, au moins l'un des atomes d'azote des formules (II) à (IV) porte éventuellement un radical alkyle en $C_1$-$C_{12}$, linéaire, ramifié ou cyclique, éventuellement substitué par au moins un groupement hydroxyle, alcoxy en $C_1$-$C_8$, amino, amino substitué par un ou plusieurs radicaux alkyle, identiques ou non, linéaires ou ramifiés en $C_1$-$C_8$ éventuellement porteur d'au moins un groupement hydroxyle.

En ce qui concerne la famille des tri-(hétéro)aryle méthanes, ces derniers correspondent plus particulièrement aux formules (VI) à (VIII) suivantes :

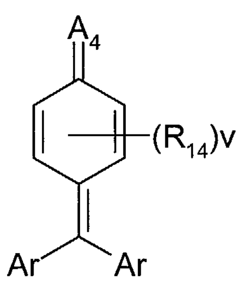

(VI)

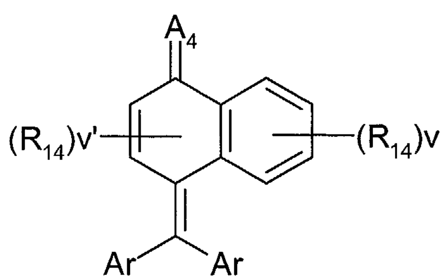

(VII)

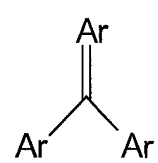

(VIII)

dans lesquelles :

- Ar, identiques ou non, représentent un radical aryle, tel que phényle ou naphtyle, éventuellement substitué ; un hétérocycle, éventuellement substitué ;
- $A_4$ représente O, N-$R_{15}$, $N^+(R_{16})_2$ dans lesquelles $R_{15}$, $R_{16}$, indépendamment les uns des autres, représentent un atome d'hydrogène, un radical alkyle, linéaire ou ramifié, en $C_1$-$C_8$ éventuellement substitué, de préférence par au moins un hydroxyle ; un groupement amido (-$CONH_2$), un groupement alcoxy en $C_1$-$C_4$ ; un radical amino ; un radical amino substitué par un ou plusieurs radicaux alkyles, identiques ou non, linéaires ou ramifiés, en $C_1$-$C_4$, éventuellement porteurs d'un groupement hydroxyle.
- $R_{14}$, identiques ou non, représentent un atome d'hydrogène ; un atome d'halogène, de préférence le chlore, le fluor ; un groupe sulfonylamino ; un hydroxyle ; un radical alkyle, linéaire ou ramifié, en $C_1$-$C_8$ éventuellement substitué ; un radical alcoxy, linéaire ou ramifié, en $C_1$-$C_8$, éventuellement substitué ; un radical alkylthio, linéaire ou ramifié, en $C_1$-$C_8$ éventuellement substitué ; un radical amino ; un radical amino substitué par un ou plusieurs radicaux alkyle, identiques ou non, linéaires ou ramifiés, en $C_1$-$C_8$, éventuellement porteurs d'au moins un groupement hydroxyle ; un radical hétérocyclique ; un groupement nitro ; un groupement cyano ; un radical aryle, de préférence en $C_6$, éventuellement substitué ; un groupement acyle ; un radical alcoxycarbonyle, linéaire ou ramifié, en $C_1$-$C_8$ un groupement carboxamido ; $-CO_2H$ ; $-SO_3H$ ; $-PO_3H_2$ ; $-PO_4H_2$ ;
- v est égal à 4 ; v' est égal à 2.

De préférence, lorsque Ar représente un hétérocycle, ce dernier peut être choisi parmi les hétérocycles, substitués ou non, de type thiophène, benzothiophène, furane, benzofurane, indole, indoline, carbazole, pyridine, déhydroquinoléine, chromone, julodinine, thiadiazole, triazole, isoxazole, oxazole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipéronyle, pipérazine, azétidine, pyrrolidine, aziridine.

De plus, les radicaux Ar peuvent être substitué plus particulièrement par un ou plusieurs atomes d'halogène, de préférence le chlore, le fluor ; un groupe sulfonylamino ; un hydroxyle ; un radical alkyle, linéaire ou ramifié, en $C_1$-$C_8$ éventuellement substitué ; un radical alcoxy, linéaire ou ramifié, en $C_1$-$C_8$, éventuellement substitué ; un radical alkylthio, linéaire ou ramifié, en $C_1$-$C_8$ éventuellement substitué ; un radical amino ; un radical amino substitué par un ou plusieurs radicaux alkyle, identiques ou non, linéaires ou ramifiés, en $C_1$-$C_8$, éventuellement porteurs d'au moins un groupement hydroxyle ; un radical hétérocyclique ; un groupement nitro ; un groupement cyano ; un radical aryle, de préférence en $C_6$, éventuellement substitué ; un groupement acyle ; un radical alcoxycarbonyle, linéaire ou ramifié, en $C_1$-$C_8$ ; un groupement carboxamido ; $-CO_2H$ ; $-SO_3H$ ; $-PO_3H_2$ ; $-PO_4H_2$.

Il est à noter que le chromophore peut être relié au bras de liaison par l'intermédiaire de l'un des groupes $A_4$, $R_{14}$, $R_{15}$, $R_{16}$, Ar, ou par l'un l'intermédiaire d'un cycle. Dans ce cas, le radical porté par le cycle représente une liaison simple entre le chromophore et le bras de liaison.

Le colorant mixte selon la présente invention peut de même comprendre au moins un chromophore de la famille des méthines.

Ces derniers sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle.

Plus particulièrement, les chromophores de cette famille sont issus de chromophores de type méthine, azométhine, mono- et di- arylméthane, indamines (ou diphénylamines), indophénols, indoanilines, carbocyanines, azacabocyanines et leurs isomères, diazacarbocyanines et leurs isomères, tétraazacarbocyanines, hémicyanines.

Plus particulièrement, parmi les chromophores de la famille des méthines, on peut citer les chromophores correspondant à la formule suivante (IX) ainsi que ses formes tautomères :

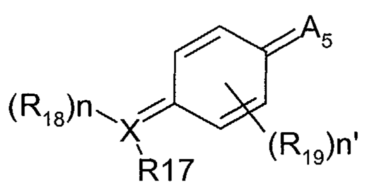

Formule (IX) dans laquelle :

- $R_{17}$, $R_{18}$, identiques ou non, représentent un atome d'hydrogène ; un radical aryle en $C_6$-$C_{30}$ un radical alkyl($C_1$-$C_8$) aryle, la partie aryle étant éventuellement substituée par un ou plusieurs groupements, identiques ou non, choisis de préférence parmi les groupements hydroxyle, alcoxy, linéaire ou ramifié, substitué ou non, en $C_1$-$C_4$, amino, amino substitué par un ou plusieurs radicaux alkyles, identiques ou non, linéaires ou ramifiés, en $C_1$-$C_4$ éventuellement porteurs d'au moins un groupement hydroxyle, les atomes d'halogène, de préférence le chlore ; un radical hétérocyclique, choisi notamment parmi les hétérocycles thiophène, furane, pipéronyle, indole, indoline, pyridine, carbazole, déhydroquinoléïne, chromone ;
- $R_{17}$, $R_{18}$, ne peuvent en outre représenter simultanément ni un radical aromatique, ni un radical hétéroaromatique ;
- $R_{19}$, identiques ou différents, représentent un atome d'hydrogène ; un radical alkyle, linéaire ou ramifié, en $C_1$-$C_8$ éventuellement substitué ; un radical aryle en $C_6$-$C_{30}$, éventuellement substitué ; un radical amino ; un radical amino substitué par ou plusieurs radicaux alkyle, linéaires ou ramifiés, en $C_1$-$C_4$, éventuellement porteurs d'au moins un groupement hydroxyle ; un groupement hydroxyle ; un radical alcoxy, linéaire ou ramifié, en $C_1$-$C_8$, éventuellement porteur d'au moins un groupement hydroxyle, alcoxy en $C_1$-$C_4$ ; un atome d'halogène et de préférence le chlore ;
- X représente un atome d'azote, un atome de carbone ;
- le coefficient n représente 0 lorsque X est un atome d'azote, et 1 lorsque X est un atome de carbone ;

- le coefficient n' est égal à 4 ;
- $A_5$ représente un groupement amino ; un groupement amino substitué par un ou plusieurs radicaux alkyles en $C_1$-$C_8$, identiques ou non, linéaires ou ramifiés, éventuellement porteurs d'au moins un groupement hydroxyle, un groupement ammonium $N^+(R_{20})_2$ ; $R_{20}$, identiques ou non, représentent un radical alkyle en $C_1$-$C_8$ éventuellement substitué ; un radical aryle en $C_6$ éventuellement substitué, de préférence par un ou plusieurs hydroxyle, atomes d'halogène, de préférence le chlore, le fluor, groupes nitro, groupes cyano, alcoxy, linéaires ou ramifiés, en $C_1$-$C_4$, monohydroxy alcoxy en $C_1$-$C_4$, linéaires ou ramifiés, polyhydroxyalcoxy, en $C_2$-$C_4$, linéaires ou ramifiés, amino substitués ou non par un ou plusieurs radicaux alkyle, hydroxyalkyle, identiques ou différents, en $C_1$-$C_4$, linéaires ou ramifiés.

Par ailleurs, le chromophore est relié au bras de liaison par l'intermédiaire du groupe $A_5$ ou de l'un des radicaux $R_{17}$, $R_{18}$, $R_{19}$ et $R_{20}$ ou directement sur le ou les cycles (hétéro)aromatique. Dans ce dernier cas, le radical $R_{19}$ concerné représente alors une liaison simple entre le chromophore et le bras de liaison.

De préférence, lorsque $R_{17}$ ou $R_{18}$ représente un radical aryle en $C_6$, ce dernier peut éventuellement être substitué, de préférence par au moins un groupement hydroxyle, au moins un groupement amino ; au moins un groupement amino substitué par un ou plusieurs radicaux alkyles en $C_1$-$C_8$, identiques ou non, éventuellement porteurs d'au moins un groupement hydroxyle ; un ou plusieurs atomes d'halogène ; au moins un radical alkylsulfonamido en $C_1$-$C_{12}$ (alkyl-$SO_2$-NH-) ; au moins un radical alkylsulfamoyle en $C_1$-$C_{12}$ (alkyl-NH-$SO_2$-) ; au moins un radical acyloxy dont la partie alkyle est en en $C_1$-$C_{12}$ ; alcoxycarbonyle dont la partie alkyle est en $C_1$-$C_{12}$ ; au moins un radical carboxyle.

Conviennent aussi les chromophores de formule suivante (X) :

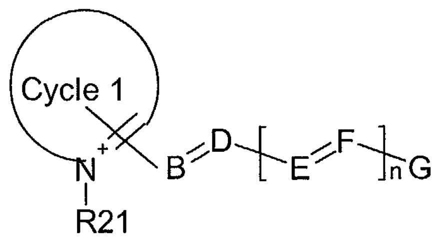

et le cas échéant leurs formes tautomères ; formule dans laquelle

B, D, E et F, identiques ou différents, représentant un atome d'azote ou un groupe C-$R_{22}$, $R_{22}$, identiques ou non, représentent un atome d'hydrogène, un radical alkyle en $C_1$-$C_8$ éventuellement substitué, de préférence par au moins un hydroxyle ; un radical alcoxy, linéaire ou ramifié, en $C_1$-$C_4$ ; un radical amino ; un radical amino substitué par un ou plusieurs radicaux alkyle, identiques ou non, linéaires ou ramifiés, en $C_1$-$C_4$, éventuellement porteurs d'au moins un groupe hydroxyle ; un radical aryle en $C_6$ éventuellement substitué, un radical hétéroaryle comprenant de 5 à 12 chaînons, éventuellement substitué ;
n = 0 ou 1 ;
G représentant un Cycle 4, ou les restes :

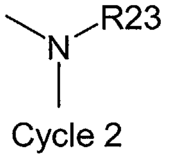
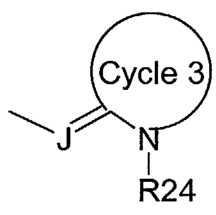

formules dans lesquelles :

$R_{21}$ et $R_{24}$ représentent indépendamment l'un de l'autre, un radical alkyle, linéaire ou ramifié, en $C_1$-$C_8$ éventuellement substitué ; un radical benzyle éventuellement substitué ;
$R_{23}$ représente un atome d'hydrogène, un radical alkyle en $C_1$-$C_8$ éventuellement substitué, de préférence par au moins un hydroxyle, un radical alcoxy, linéaire ou ramifié, en $C_1$-$C_4$, un radical amino, un radical amino substitué

par un ou plusieurs radicaux alkyle, identiques ou non, linéaires ou ramifiés, en $C_1$-$C_4$, éventuellement porteurs d'au moins un groupe hydroxyle ; un radical aryle en $C_6$ éventuellement substitué ; un radical hétéroaryle en $C_2$-$C_{12}$ éventuellement substitué ;

**J** représente un atome d'azote ou un groupe C-$R_{25}$ ; avec $R_{25}$ ayant la même signification que $R_{22}$ ;

**Cycle 1** étant choisi parmi les radicaux hétéroaromatiques à 5 à 12 chaînons, portant au moins une charge cationique sur un atome d'azote et comprenant éventuellement au moins un autre hétéroatome choisi parmi l'azote, l'oxygène et/ou le soufre ; lesdits radicaux étant éventuellement substitués par au moins un radical alkyle, linéaire ou ramifié, en $C_1$-$C_8$ substitué ou non ; au moins un radical alcoxy, linéaire ou ramifié, en $C_1$-$C_8$ substitué ou non ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical aromatique en $C_5$-$C_6$ ; au moins un groupement hydroxyle ; alcoxycarbonyle ; nitro ; cyano ; alkylsulfonamido en $C_1$-$C_{12}$ (alkyl-$SO_2$-NH-) ; alkylsulfamoyle en $C_1$-$C_{12}$ (alkyl-NH-$SO_2$-) ;

**Cycle 2** étant choisi parmi les radicaux aromatiques en $C_6$-$C_{12}$ ; les radicaux hétéroaromatiques à 5 à 12 chaînons, comprenant au moins un hétéroatome choisi parmi l'azote, l'oxygène et/ou le soufre ; lesdits radicaux étant éventuellement substitués par au moins un radical alkyle, linéaire ou ramifié, en $C_1$-$C_8$ ; au moins un radical alcoxy, linéaire ou ramifié, en $C_1$-$C_8$ ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical (hétéro)aromatique comprenant de préférence 5 à 6 chaînons ; au moins un groupement hydroxyle. De préférence Cycle 2 représente un radical aromatique en $C_6$-$C_{30}$, éventuellement substitué comme indiqué ci-dessus ;

**Cycle 3** étant choisi parmi les radicaux hétéroaromatiques à 5 ou 6 chaînons comprenant au moins un hétéroatome choisi parmi l'azote, l'oxygène et/ou le soufre ; lesdits radicaux étant éventuellement substitués par au moins un radical alkyle, linéaire ou ramifié, en $C_1$-$C_8$ ; au moins un radical alcoxy, linéaire ou ramifié, en $C_1$-$C_8$ ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical aromatique en $C_5$-$C_6$ ; au moins un groupement hydroxyle ; alcoxycarbonyle ; nitro ; cyano ; alkylsulfonamido en $C_1$-$C_{12}$ (alkyl-$SO_2$-NH-) ; alkylsulfamoyle en $C_1$-$C_{12}$ (alkyl-NH-$SO_2$-) ;

**Cycle 4** étant choisi parmi les radicaux aromatiques en $C_6$-$C_{12}$ ; les radicaux hétéroaromatiques à 5 à 12 chaînons, comprenant au moins un hétéroatome choisi parmi l'azote, l'oxygène et/ou le soufre ; lesdits radicaux étant éventuellement substitués par au moins un radical alkyle, linéaire ou ramifié, en $C_1$-$C_8$ ; au moins un radical alcoxy, linéaire ou ramifié, en $C_1$-$C_8$ ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical (hétéro)aromatique comprenant de préférence 5 à 6 chaînons; au moins un groupement hydroxyle ;

à la condition que lorsque n vaut 1 et G représente un cycle, alors B, D, E et F ne peuvent représenter simultanément un atome d'azote ; et que lorsque n vaut 0 et G représente un cycle, alors B et D ne représentent pas simultanément un atome d'azote.

Par ailleurs, le chromophore est relié au bras de liaison par l'intermédiaire de l'un des radicaux $R_{21}$, $R_{22}$, $R_{23}$, $R_{24}$, et $R_{25}$ ou des cycles. Dans ce cas, le radical porté par le cycle représente une liaison simple entre le chromophore et le bras de liaison.

La formule (X) comprend les isomères de position correspondant aux différentes possibilités d'implantation de la liaison de B sur le cycle 1 par rapport à l'atome d'azote quaternisé).

Selon un mode de réalisation particulièrement avantageux de l'invention, le chromophore de la famille des méthines est choisi parmi les composés de formules suivantes, ainsi que leurs formes tautomères :

| | |
|---|---|
| Indamines (Diphénylamines) | 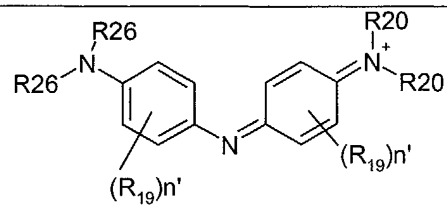 |
| Diphénylméthanes | 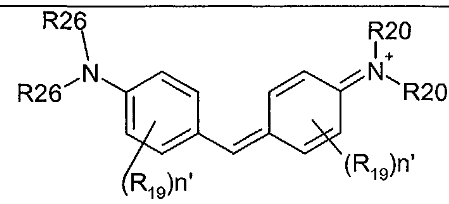 |
| Indoaniline | 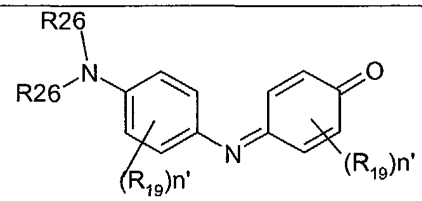 |
| Indophénol | 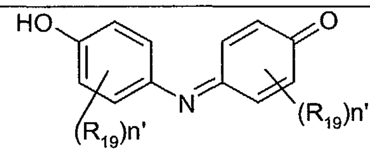 |
| Carbocyanine | 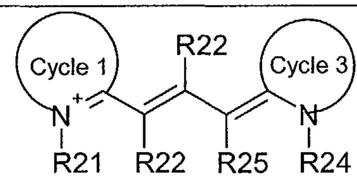 |
| Azacarbocyanine | 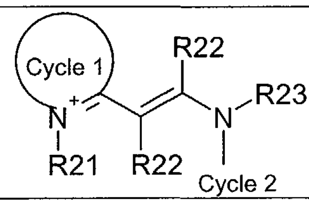 |
| Isomère azacarbocyanine | 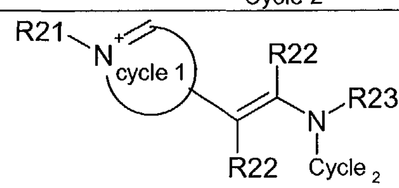 |
| Diazacarbocyanine | 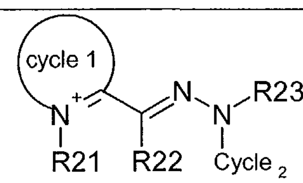 |
| Isomère diazacarbocyanine | 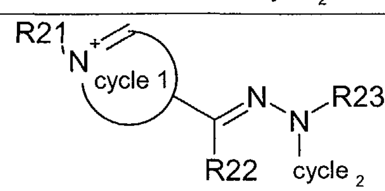 |

| | |
|---|---|
| Tétraazacarbocyanine | 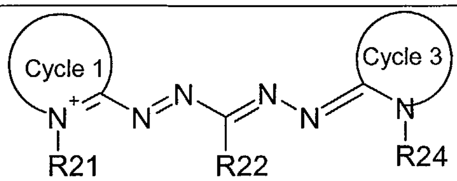 |
| Hémicyanine | 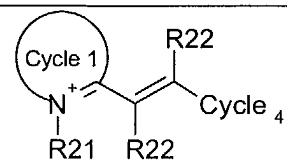 |

Formules dans lesquelles :

$R_{26}$ identiques ou non, représentent un atome d'hydrogène, un radical alkyle en $C_1$-$C_8$ éventuellement substitué ; un radical aryle en $C_6$ éventuellement substitué, de préférence par un ou plusieurs hydroxyle, atomes d'halogène, de préférence le chlore, le fluor, groupes nitro, groupes cyano, alcoxy, linéaires ou ramifiés, en $C_1$-$C_4$, monohydroxy alcoxy en $C_1$-$C_4$, linéaires ou ramifiés, polyhydroxyalcoxy, en $C_2$-$C_4$, linéaires ou ramifiés, amino substitués ou non par un ou plusieurs radicaux alkyle, hydroxyalkyle, identiques ou différents, en $C_1$-$C_4$, linéaires ou ramifiés ;
Les groupements et radicaux $R_{20}$, $R_{21}$, $R_{22}$, $R_{23}$, $R_{24}$, $R_{25}$, $R_{26}$, $Cycle_1$, $Cycle_2$, $Cycle_3$ et $Cycle_4$ étant définis comme précédemment.
De préférence, le groupement $Cycle_1$ représente un cycle imidazolium, pyridinium ou indolinium, éventuellement substitué comme indiqué auparavant.
De préférence, le groupement $Cycle_3$ représente un cycle imidazole, pyridine ou indoline, éventuellement substitué comme indiqué auparavant.
De préférence, le groupement $Cycle_2$ représente un radical aromatique en $C_6$ éventuellement substitué comme indiqué auparavant.
De préférence, le groupement $Cycle_4$ représente un radical aromatique en $C_6$ éventuellement substitué comme indiqué auparavant.

Précisons que le chromophore peut être relié au bras de liaison par l'intermédiaire de l'un des groupes $R_{19}$, $R_{20}$, $R_{21}$, $R_{22}$, $R_{23}$, $R_{24}$ et $R_{25}$ ou par l'intermédiaire d'un cycle aromatique ou hétéroaromatique. Dans le cas de rattachement par l'intermédiaire d'un cycle, le radical porté sur ledit cycle représente une liaison simple entre le chromophore et le bras de liaison.
Conformément à un mode de réalisation particulier de l'invention, le chromophore est choisi parmi les composés de formule (X) et de préférence parmi les diazacarbocynaines et leurs isomères, les hémicyanines. Plus particulièrement, $R_{22}$, identiques ou non, représentent un atome d'hydrogène, un radical alkyle en $C_1$-$C_8$ éventuellement substitué, de préférence par au moins un hydroxyle ; un radical aryle en $C_6$ éventuellement substitué ; n = 0 ; G représentant un Cycle4, ou -N($R_{23}$)-Cycle2 avec $R_{23}$ représentant un atome d'hydrogène, un radical alkyle en $C_1$-$C_8$ éventuellement substitué, de préférence par au moins un hydroxyle ; les cycle2 et cycle4, identiques ou non, représentant un radical aromatique en $C_6$, éventuellement substitués.
Selon ce mode de réalisation particulier de l'invention, le chromophore et le bras de liaison sont reliés par l'intermédiaire du radical $R_{21}$.
Il est à noter que les chromophores décrits ci-dessus peuvent être préparés selon l'enseignement des brevets et demandes de brevets suivants GB 822846 ; DE 1254118 ; GB 1047796 ; US 3652556 ; US 3423427 ; BE 702239 ; GB 702240 ; US 3995088 ; US 4054718 ; US 4670385 ; US 5094688 ; US 5097034.
Le colorant mixte peut de même comprendre au moins un chromophore de la famille des carbonyles.
Parmi les chromophores de ce type, on peut citer par exemple les chromophores issus des colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.
Plus particulièrement, le chromophore de la famille des carbonyles est représenté par la formule suivante (XI) :

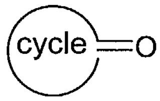

Formule dans laquelle le cycle représente un cycle à 5 ou 6 chaînons, dont éventuellement au moins l'un d'entre eux est remplacé par un hétéroatome choisi parmi l'oxygène, l'azote, le soufre, ou par une fonction carbonyle additionnelle ; ledit cycle étant éventuellement substitué par un radical ou plusieurs radicaux choisis parmi les radicaux alkyle, linéaire ou ramifié, en $C_1$-$C_8$, éventuellement substitués ; par un ou plusieurs radicaux hydroxyle ; les atomes d'halogène comme de préférence le chlore ; les groupements nitro, cyano, amino, alkylamino ; ledit cycle étant éventuellement condensé avec un ou plusieurs cycles aromatiques en $C_6$, ce ou ces cycles pouvant eux-mêmes être condensés à un ou plusieurs cycles aromatiques dont éventuellement au moins l'un des atomes de carbone est remplacé par un ou plusieurs hétéroatomes choisis parmi l'oxygène, l'azote ou le soufre.

Par ailleurs, le chromophore étant relié au bras de liaison par l'intermédiaire de l'un des radicaux substituants les cycles ou par l'intermédiaire d'un cycle. Dans ce dernier cas, le radical porté par le cycle représente une liaison simple entre le chromophore et le bras de liaison.

Conformément à un mode de réalisation préféré de l'invention, le chromophore de la famille des carbonyles est représenté par l'une des formules suivantes, ainsi que les formules tautomères :

| | |
|---|---|
| Acridones | 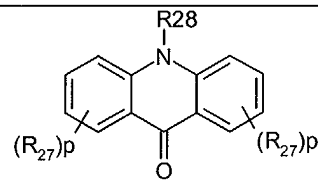 |
| Anthraquinones | 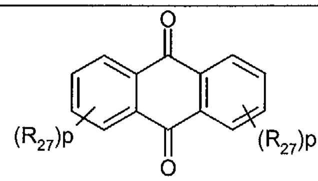 |
| Benzanthrones | 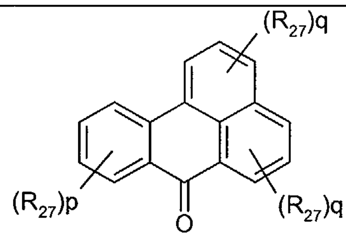 |
| Benzoquinones | 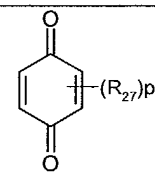 |
| Flavones | 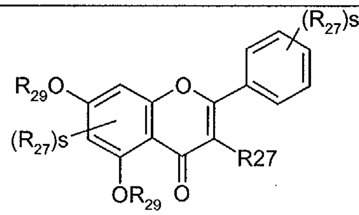 |
| Indanthrones | 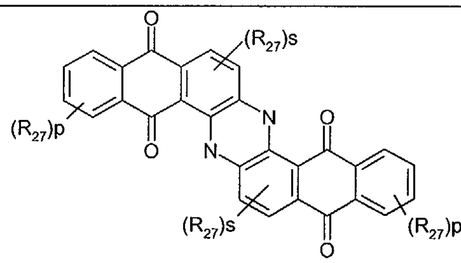 |
| Naphthoquinones | 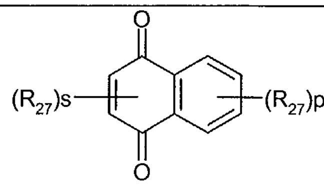 |
| Quinacridones | 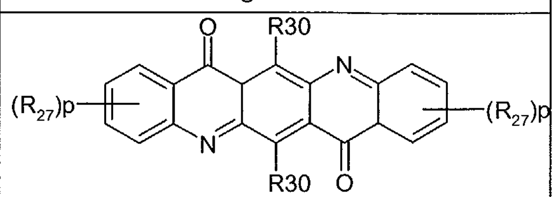 |

| | |
|---|---|
| Indigoïdes | 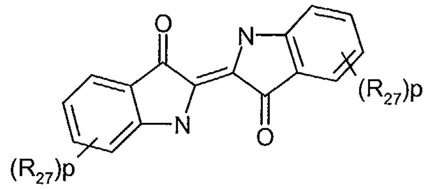 |
| Thioindigos | 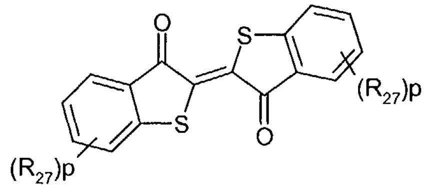 |
| Naphthalimides | 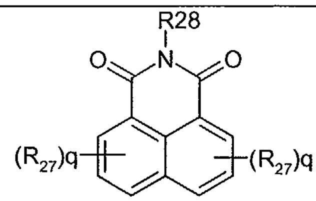 |
| Dicétopyrrolopyrroles | 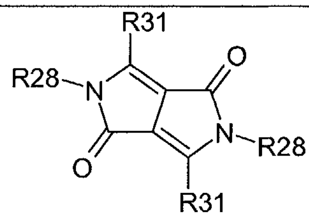 |
| Coumarines | 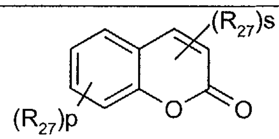 |

Formules dans lesquelles :

$R_{27}$, $R_{28}$, $R_{30}$, $R_{31}$, identiques ou non, représentent un atome d'hydrogène ; un radical alkyle, linéaire ou ramifié, en $C_1$-$C_8$, éventuellement substitué ; un groupement hydroxyle ; un radical alcoxy, linéaire ou ramifié, en $C_1$-$C_8$ ; un radical amino ; un radical amino substitué par un ou plusieurs radicaux alkyle, identiques ou non, linéaires ou ramifiés, en $C_1$-$C_8$, éventuellement porteurs d'au moins un groupement hydroxyle ; un atome d'halogène et de préférence le chlore ou le fluor ; un groupement nitro ; un groupement cyano ;
$R_{29}$ représente un atome d'hydrogène, un radical alkyle, linéaire ou ramifié, en $C_1$-$C_8$ ; éventuellement substitué ;
$R_{31}$, identiques ou non, représentent un radical aryle en $C_6$, éventuellement substitué, de préférence par au moins un hydroxyle, au moins un amino, au moins un amino substitué par un ou plusieurs radicaux alkyles, identiques ou non, linéaires ou ramifiés en $C_1$-$C_8$ éventuellement porteurs d'au moins un hydroxyle, au moins un groupe alcoxy, linéaire ou ramifié, en $C_1$-$C_8$ éventuellement porteur d'au moins un hydroxyle, au moins un atome d'halogène de préférence le chlore, le fluor, au moins un groupement nitro, au moins un groupement cyano ;
p est égal à 4 ; q est égal à 3 ; r est égal à 5; s est égal à 2.

Le chromophore peut être relié au bras de liaison par l'intermédiaire de l'un des radicaux $R_{27}$, $R_{28}$, $R_{29}$, $R_{30}$, $R_{31}$ ou par l'intermédiaire de l'un des cycles aromatiques ou hétéroaromatiques. Dans ce cas, le radical porté par le cycle représente une liaison simple entre le chromophore et le bras de liaison.

Le colorant mixte de la présente invention peut de plus comprendre au moins un chromophore de la famille des azines cycliques.

Plus particulièrement, le chromophore de la famille des azines cycliques est choisi parmi les radicaux issus des colorants choisi parmi les azine, xanthène, thioxanthène, fluorindine, acridine, (di)oxazine, (di)thiazine, pyronine.

Parmi les chromophores de la famille des azines cycliques, on peut citer les composés de formule suivante (XII) ainsi que ses formes tautomères :

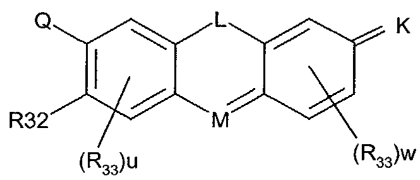

formule dans laquelle :

L, représente un hétéroatome de préférence choisi parmi l'oxygène, le soufre ; un groupe NH ; un groupe N-$R_{34}$, M représente un hétéroatome de préférence choisi parmi l'oxygène, le soufre, l'azote; un groupe $N^+$-$R_{34}$ ; un groupe CH ; un groupe C-$R_{35}$ ;

Q, K, identiques ou non, représentent un groupement hydroxyle ; un radical amino ; un radical amino substitué par un ou plusieurs radicaux alkyle, identiques ou non, linéaires ou ramifiés, en $C_1$-$C_8$, éventuellement porteurs d'au moins un groupement hydroxyle, un radical aryle éventuellement substitué, un radical alkyl($C_1$-$C_8$)aryle éventuellement substitué ; un groupement ammonium de type N+$(R_{36})_t$ avec t égal à 2 pour K et à 3 pour Q, $R_{36}$, identiques ou non, représentent un atome d'hydrogène ; un radical alkyle, linéaire ou ramifié, en $C_1$-$C_8$, éventuellement porteur d'au moins un groupement hydroxyle ; un radical aryle éventuellement substitué, un radical alkyl ($C_1$-$C_8$)aryle la partie aryle étant éventuellement substituée ; un radical alkyle en C1-C8, linéaire ou ramifiée, éventuellement substituée, un radical alcoxy, linéaire ou ramifié, en $C_1$-$C_8$, éventuellement substitué ;

A la condition que Q et K ne représentent pas à la fois un groupement ammonium de type $N^+(R_{36})_t$ ;

$R_{32}$, $R_{33}$, identiques ou non, représentent un atome d'hydrogène ; un radical alkyle, linéaire ou ramifié, en $C_1$-$C_8$ éventuellement substitué ; un radical amino ; un radical amino substitué ou non par un ou plusieurs radicaux, identiques ou non, choisis parmi les radicaux alkyle, linéaires ou ramifiés, en $C_1$-$C_8$ éventuellement substitués, les radicaux phényle éventuellement substitués ; un atome d'halogène de préférence le chlore, le fluor ;

Au cas où Q représente un radical amino substitué ou non, ou un groupement hydroxyle, $R_{32}$ peut représenter un radical alkylamino ou alcoxy formant avec l'atome d'azote ou d'oxygène du radical **J**, un cycle à 6 chaînons, éventuellement condensé avec un radical aromatique, ledit radical aromatique étant éventuellement substitué par au moins un groupement amino ou amino substitué ou non par un ou plusieurs radicaux identiques ou non, choisis parmi les radicaux alkyle, linéaires ou ramifiés, en $C_1$-$C_8$ éventuellement substitués, les radicaux phényle éventuellement substitués ;

$R_{34}$, $R_{35}$, identiques ou non, représentent un radical alkyle, linéaire ou ramifié en $C_1$-$C_8$ éventuellement substitué, de préférence par au moins un hydroxyle ; au moins un radical alcoxy, linéaire ou ramifié en $C_1$-$C_8$ ; au moins un radical amino ; au moins un radical amino substitué par un ou plusieurs radicaux alkyles, identiques ou non, linéaires ou ramifiés, en $C_1$-$C_8$, éventuellement porteurs d'au moins un radical hydroxyle.

$R_{34}$, $R_{35}$, identiques ou non, peuvent aussi représenter un radical aryle, de préférence en $C_6$, éventuellement substitué, de préférence par au moins un radical alkyle, linéaire ou ramifié, en $C_1$-$C_8$ ; par au moins un groupement hydroxyle ; par au moins un radical alcoxy, linéaire ou ramifié, en $C_1$-$C_8$ ; par au moins un radical amino ; par au moins un radical amino substitué par un ou plusieurs radicaux alkyle, identiques ou non, linéaires ou ramifiés, en $C_1$-$C_8$, éventuellement porteurs d'au moins un groupement hydroxyle ; un atome d'halogène et de préférence le chlore ou le fluor ; par au moins un groupement nitro ; par au moins un groupement cyano. De préférence $R_{34}$ et $R_{35}$, identiques ou non, représentent un radical alkyle, linéaire en $C_1$-$C_4$, un radical aryle éventuellement substitué ;

Par ailleurs, le chromophore est relié plus particulièrement au bras de liaison par l'intermédiaire de l'un des radicaux $R_{32}$, $R_{33}$, $R_{34}$, $R_{35}$, $R_{36}$, ou par l'intermédiaire d'un cycle. Dans ce cas, le radical porté par le cycle représente une liaison simple entre le chromophore et le bras de liaison.

Conformément à un mode de réalisation préféré, le chromophore de la famille des azines cycliques est représenté par les formules ci-dessous, ainsi que, le cas échéant, leurs formes tautomères :

| | |
|---|---|
| Xanthènes | 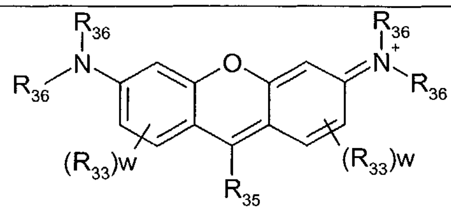 |
| Thioxanthènes | 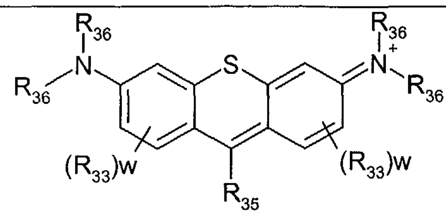 |
| Acridines | 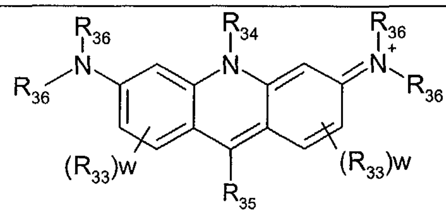 |
| Oxazines | 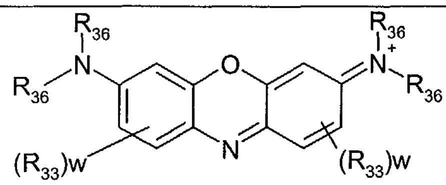 |
| Dioxazines | 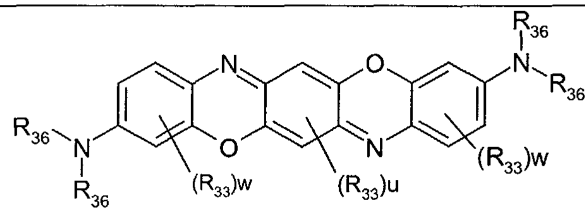 |
| Thiazines | 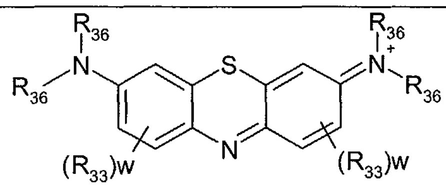 |
| Phénazines | 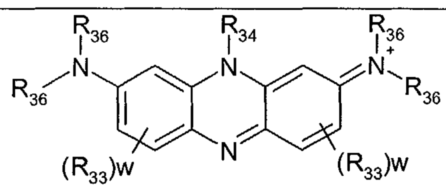 |

Formules dans lesquelles les radicaux et les coefficients u et w ont été définis précédemment.

Le chromophore est de plus relié au bras de liaison par l'intermédiaire de l'un des radicaux $R_{33}$, $R_{34}$, $R_{35}$, $R_{36}$ ou par l'intermédiaire d'un cycle. Dans ce cas, le radical porté par le cycle représente une liaison simple entre le chromophore et le bras de liaison.

A titre de chromophore possible, le colorant mixte peut comprendre au moins un chromophore de la famille des composés nitrés (hétéro)aromatiques.

Plus particulièrement, on peut citer par exemple les composés correspondant aux formules (XIII) et (XIV) ci-dessous, ainsi que leurs formes tautomères :

| | |
|---|---|
| benzénique | 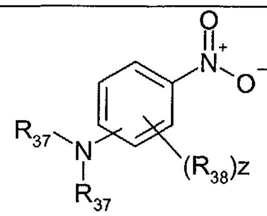 (XIII) |
| Pyridinique | 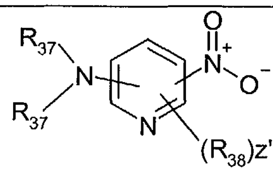 (XIV) |

Formules dans lesquelles

$R_{37}$, identiques ou non, représentent un atome d'hydrogène ; un radical alkyle, linéaire ou ramifié, en $C_1$-$C_8$, éventuellement porteur d'au moins un groupement hydroxyle ; un radical aryle éventuellement substitué, un radical alkyl($C_1$-$C_8$)aryle la partie aryle étant éventuellement substituée ; un radical alkyle en C1-C8, linéaire ou ramifiée, éventuellement substituée, un radical alcoxy, linéaire ou ramifié, en $C_1$-$C_8$, éventuellement substitué ;

$R_{38}$, identiques ou différents, représentent un atome d'hydrogène ; alkyle linéaire ou ramifié en $C_1$-$C_4$, éventuellement substitué ; un radical alcoxy linéaire ou ramifié en $C_1$-$C_8$, éventuellement substitué ; un radical aryle en $C_6$ éventuellement substitué ; un radical amino ; un radical amino substitué par un ou plusieurs radicaux choisis parmi les radicaux alkyle, identiques ou non, linéaires ou ramifiés, en $C_1$-$C_8$, de préférence en $C_1$-$C_4$, éventuellement substitués, de préférence par au moins un groupe hydroxyle, alcoxy, linéaire ou ramifié en $C_1$-$C_4$, thioalkyl linéaires ou ramifiés en $C_1$-$C_4$, alkylsulfonamido linéaires ou ramifiés en $C_1$-$C_4$, parmi les radicaux aryle en $C_6$ éventuellement substitué et parmi les radicaux hétéroaryle comprenant 5 à 6 chaînons éventuellement substitués ; un groupe hydroxyle ; un groupe nitro ; un groupe cyano ;

Le coefficient z étant égal à 4 et le coefficient z' étant égal à 3.

En outre, le chromophore est relié au bras de liaison par l'intermédiaire de $R_{37}$, $R_{38}$, ou par l'intermédiaire du cycle. Dans ce cas, le radical porté par le cycle représente une liaison simple entre le chromophore et le bras de liaison.

Ainsi, à titre d'exemples de colorants mixtes comprenant deux chromophores, on peut citer par exemple les colorants mixtes suivants :

Azo - bras de liaison - azine
Azo - bras de liaison - méthine
Azo - bras de liaison - carbonyle
Azo - bras de liaison - nitré
Azo - bras de liaison - tri- (hétéro)aryle méthane
tri- (hétéro)aryle méthane - bras de liaison - azine
tri- (hétéro)aryle méthane - bras de liaison - méthine
tri- (hétéro)aryle méthane - bras de liaison - carbonyle
tri- (hétéro)aryle méthane - bras de liaison - nitré
tri- (hétéro)aryle méthane - bras de liaison - tri- (hétéro)aryle méthane

Comme indiqué précédemment, les chromophores du colorant mixte sont reliés entre eux au moyen d'au moins un bras de liaison stoppant la délocalisation des électrons de chacun des chromophores.

Ainsi, le bras de liaison comprend un atome ou un groupe d'atomes qui isole(nt) chacun des chromophores du colorant mixte.

De préférence, la liaison entre le radical et le bras de liaison est réalisée par l'intermédiaire d'un atome d'azote ou d'oxygène.

Il est à noter en outre, que le bras de liaison peut être cationique ou non cationique.

De plus, le bras de liaison peut être divalent, trivalent ou tétravalent.

Selon un mode de réalisation avantageux de l'invention, le bras de liaison est choisi parmi une chaîne hydrocarbonée en $C_1$-$C_{20}$, de préférence en $C_1$-$C_{12}$, linéaire ou ramifiée, de préférence une chaîne alkyle, un ou plusieurs des atomes de carbone de ladite chaîne pouvant chacun être remplacés par un hétéroatome tel que le soufre, l'azote,

l'oxygène, à la condition que la chaîne ne comprenne pas deux hétéroatomes adjacents ; par un hétérocycle comprenant 5 ou 6 chaînons, saturé ou non, comprenant de préférence au moins deux atomes d'azote ; ladite chaîne hydrocarbonée pouvant être insaturée ou contenir un radical arylène ; parmi un radical arylène ; parmi un radical divalent téréphtalamide ; parmi un radical divalent ou trivalent, par exemple de type triazine.

De préférence, le bras de liaison correspond à une chaîne alkyle en $C_1$-$C_{20}$, de préférence en $C_1$-$C_{12}$, linéaire ou ramifiée, dont au moins l'un des atomes de carbone peut être remplacé par un hétérocycle comprenant 5 ou 6 chaînons, saturé ou non, comprenant de préférence au moins deux atomes d'azote.

Les colorants mixtes de l'invention peuvent être préparés selon des réactions chimiques connues en soi, à partir de chromophores fonctionnalisés capables de réagir avec le bras de liaison choisi.

Par exemple, lorsque le bras de liaison est un groupe triazine alors le chromophore comporte un groupe réactif amino, OH ou SH, et la synthèse s'effectue, par exemple, selon les schémas ci dessous :

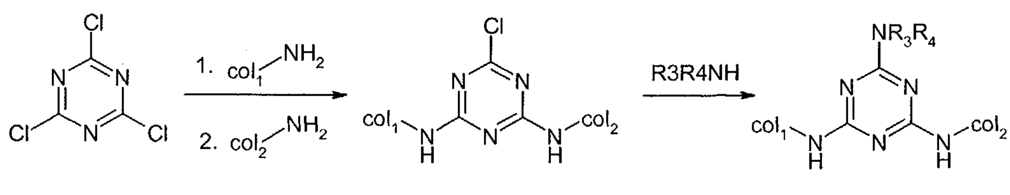


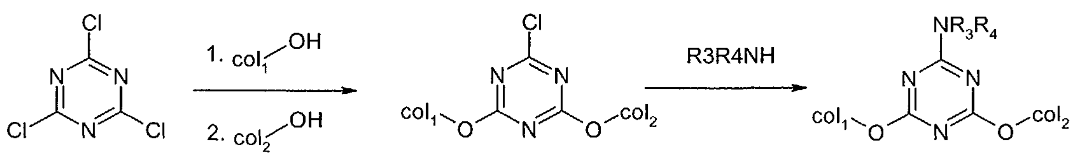


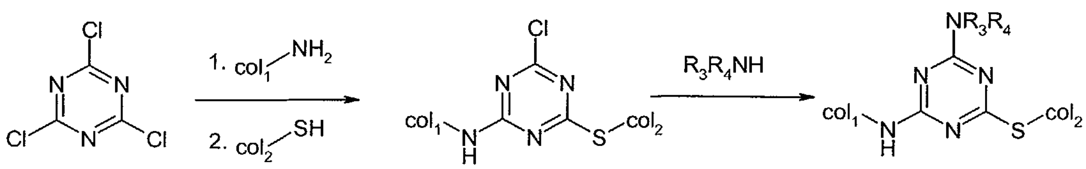


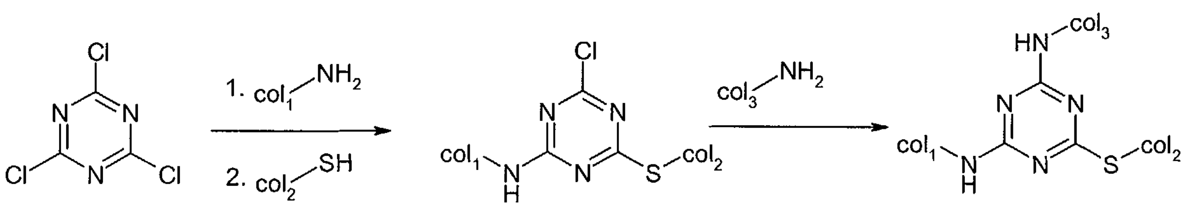


Selon une première étape, un premier chromophore est mélangé au composé susceptible de former le bras de liaison, par exemple la trichlorotriazine. Lorsque cette réaction est terminée, on ajoute au milieu réactionnel un second chromophore. Cette séquence peut être répétée autant de fois qu'il y a de groupes réactifs sur le composé susceptible de former le bras de liaison.

Pour la préparation d'un colorant mixte Col1-bras liaison-Col 2, le rapport molaire du bras de liaison par rapport au colorant 1 est généralement compris entre 10:1 et 0,5:1, de préférence égal à 1:1. Ce rapport peut être modifié lorsqu'on utilise plus d'un bras de liaison ou plusieurs chromophores.

La température de réaction est en général comprise entre -100°C et +130°C, de préférence entre -5°C et 100°C. Le temps de réaction dépend de la réactivité des espèces en présence et de la température de réaction. En général, le temps de réaction est compris entre 10 minutes et 8 heures, de préférence entre 30 minutes et 4 heures.

Avantageusement, le pH du mélange réactionnel est compris entre 2 et 12.

La réaction peut en outre être conduite dans de l'eau et/ou dans un ou plusieurs solvants organiques.

Plusieurs publications décrivent la réaction d'association chimique entre deux chromophores identiques. On peut citer par exemple les documents ISBN 0901956759, WO 0278596, DE19845640, US 5708151.

En outre, les réactions ou les réactions d'un bras de liaison avec deux composés différents, colorants ou non,

ont été décrites dans la littérature, par exemple dans WO03/029359, DE3335956, WO03/30909, WO0318021, Journal of medicinal chemistry 43(9), 2000, 1892-97 ; Chemiker Zeitung 117(7-8), 1987, 241-5.

Selon une autre possibilité, le colorant mixte peut être obtenu en suivant le schéma réactionnel suivant :

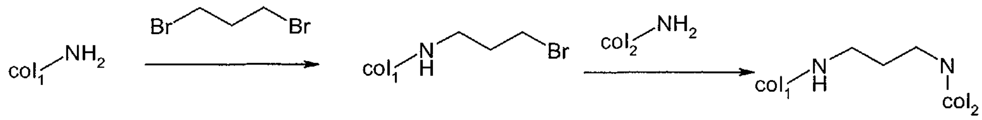

Selon une première étape, un premier chromophore est mélangé au composé susceptible de former le bras de liaison, par exemple le dibromopropane. Lorsque cette réaction est terminée, on ajoute au milieu réactionnel un second chromophore. Cette séquence peut être répétée autant de fois qu'il y a de groupes réactifs sur le composé susceptible de former le bras de liaison.

Pour la préparation d'un colorant mixte Col1-bras de liaison-Col 2, le rapport molaire du bras de liaison par rapport au colorant 1 est généralement compris entre 10:1 et 0,1:1, de préférence égal à 0,5:1. Ce rapport peut être modifié lorsqu'on utilise plus d'un bras de liaison ou plusieurs chromophores.

La température de réaction est en général comprise entre -100C et +130°C, de préférence entre -5°C et 100°C. Le temps de réaction dépend de la réactivité des espèces en présence et de la température de réaction. En général, le temps de réaction est compris entre 10 minutes et 24 heures, de préférence entre 30 minutes et 4 heures.

La réaction peut être conduite dans de l'eau et/ou dans un ou plusieurs solvants organiques.

Avantageusement, le pH du mélange réactionnel est compris entre 2 et 12.

La composition selon l'invention comprend habituellement une teneur en colorant mixte variant de 0,001 à 20% en poids, plus particulièrement, de 0,005 à 10% en poids, et de préférence, de 0,01 et 5% en poids, par rapport au poids total de la composition.

De préférence, la composition tinctoriale selon l'invention ne comprend pas de colorant mixte choisi parmi les composés suivants :

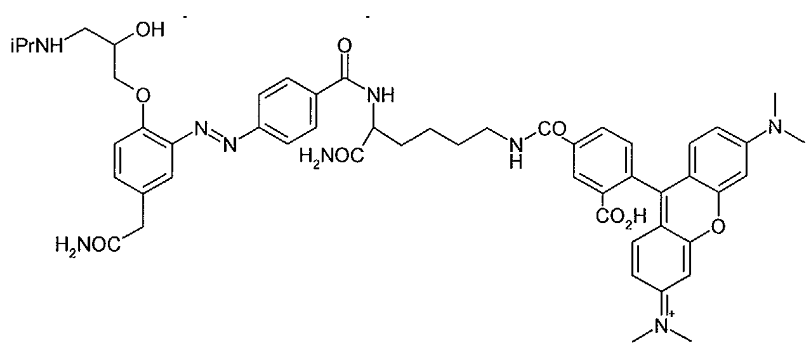

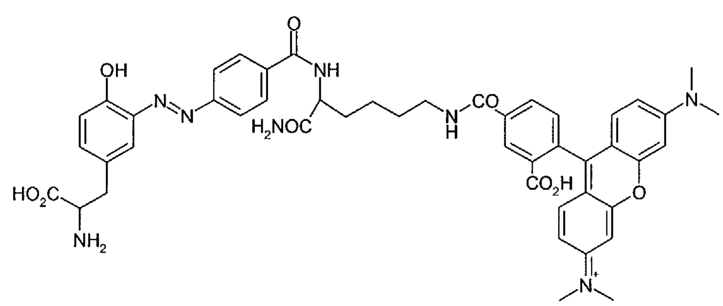

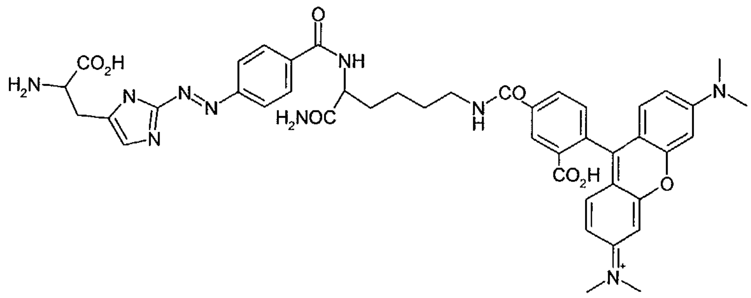

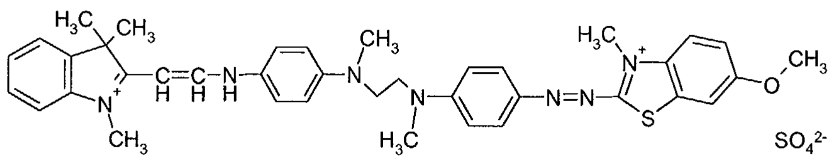

La composition tinctoriale selon l'invention peut contenir un ou plusieurs colorants directs additionnels différents du colorant mixte décrit auparavant.

On peut utiliser les colorants directs classiquement mis en oeuvre dans le domaine de la coloration des fibres kératiniques, et notamment des fibres kératiniques humaines.

A ce titre, on peut notamment citer les colorants nitrés de la série benzénique, les colorants additionnels directs azoïques, les colorants directs méthiniques. Ces colorants directs peuvent être de nature non ionique, anionique ou cationique. De préférence ces colorants directs additionnels sont de nature cationique.

Selon un mode de réalisation particulier de l'invention, la composition tinctoriale comprend une teneur en chacun des colorants directs variant de 0,001 à 10 % en poids par rapport au poids total de la composition tinctoriale.

La composition tinctoriale de l'invention peut aussi contenir une ou plusieurs bases d'oxydation et/ou un ou plusieurs coupleurs, conventionnellement utilisés pour la teinture de fibres kératiniques, et notamment des fibres kératiniques humaines.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition.

La ou les bases d'oxydation présentes dans la composition de l'invention sont en général utilisées chacune en quantité variant de 0,001 à 10 % en poids par rapport au poids total de la composition tinctoriale, de préférence variant de 0,005 à 6 % en poids.

Parmi les coupleurs utilisables, on peut notamment citer les métaphénylènediamines, les méta-aminophénols, les méta-diphénols, les coupleurs naphtaléniques, les coupleurs hétérocycliques et leurs sels d'addition.

Dans la composition de la présente invention, le ou les coupleurs sont chacun généralement présents en quantité variant de 0,001 à 10 % en poids par rapport au poids total de la composition tinctoriale, de préférence variant de 0,005 à 6 % en poids.

D'une manière générale, les sels d'addition, notamment des bases d'oxydation et des coupleurs utilisables dans le cadre de l'invention, sont par exemple choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique pour solubiliser les composés qui ne seraient pas suffisamment solubles dans l'eau.

A titre de solvant organique, on peut par exemple citer les monoalcools, linéaires ou ramifiés, de préférence saturés, comprenant 2 à 10 atomes de carbone, tels que l'alcool éthylique, l'alcool isopropylique ; les alcools aromatiques tels que l'alcool benzylique, l'alcool phényléthylique ; les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol, l'hexylèneglycol (2-méthyl 2,4-pentanediol), le néopentylglycol et le 3-méthyl-1,5-pentanediol ; ainsi que les alkyléthers de diéthylèneglycol, notamment en $C_1$-$C_4$, comme par exemple, le monoéthyléther ou le monobutyléther du diéthylèneglycol, seuls ou en

mélange.

Les solvants sont, de préférence, présents dans des proportions allant de 1 à 40 % en poids environ par rapport au poids total de la composition tinctoriale, et encore plus préférentiellement de 5 à 30 % en poids environ.

La composition tinctoriale conforme à l'invention peut également renfermer divers adjuvants utilisés classiquement dans les compositions pour la teinture des fibres kératiniques, notamment humaines et en particulier les cheveux, tels que des agents tensioactifs anioniques, cationiques, non-ioniques, amphotères, zwitterioniques ou leurs mélanges ; des polymères anioniques, cationiques, non ioniques, amphotères, zwitterioniques ou leurs mélanges ; des agents épaississants minéraux ou organiques, et en particulier les épaississants associatifs polymériques anioniques, cationiques, non ioniques et amphotères ; des agents antioxydants ; des agents de pénétration ; des agents séquestrants ; des parfums ; des tampons ; des agents dispersants ; des agents de conditionnement tels que par exemple des silicones volatiles ou non volatiles, modifiées ou non modifiées ; des agents filmogènes ; des céramides ou pseudo-céramides ; des agents conservateurs ; des agents opacifiants, etc.

Les adjuvants ci dessus sont en général présents en quantité variant, pour chacun d'eux, de 0,01 à 20 % en poids par rapport au poids de la composition.

La composition de l'invention peut en outre contenir au moins un agent oxydant.

Les agents oxydants classiquement utilisés pour la teinture d'oxydation des fibres kératiniques, notamment humaines, sont par exemple le peroxyde d'hydrogène, le peroxyde d'urée, les bromates ou ferricyanures de métaux alcalins, les persels tels que les perborates et les persulfates de métaux alcalins ou alcalino-terreux, comme le sodium, le potassium, le magnésium, seuls ou en mélanges, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition de l'invention peut de plus comprendre au moins un agent alcalin qui peut être choisi parmi ceux utilisés classiquement dans le domaine cosmétique.

Parmi ces agents alcalins, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante :

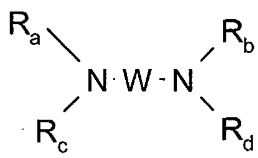
(A)

dans laquelle W est un reste propylène éventuellement substitué par un groupement hydroxyle ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

Le pH de la composition tinctoriale de l'invention est de préférence compris entre 8 et 11.

Bien entendu, l'homme de l'art veillera à choisir ce ou ces éventuels composés complémentaires de manière telle que les propriétés avantageuses attachées intrinsèquement à la composition selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, notamment humaines, et plus particulièrement des cheveux.

Le procédé de la présente invention est un procédé dans lequel on applique sur les fibres, sèches ou non, la composition selon la présente invention telle que définie précédemment.

Selon une première variante, la composition appliquée sur les fibres kératiniques, ne comprend pas d'agent oxydant. Cette variante est particulièrement appropriée lorsque la composition tinctoriale comprend au moins un colorant mixte selon l'invention et éventuellement au moins un colorant direct additionnel.

Selon une deuxième variante de l'invention, le procédé est mis en oeuvre avec au moins un agent oxydant. Cette deuxième variante est appropriée quelle que soit la nature des colorants présents (colorant mixte, colorant direct additionnel, bases d'oxydation et/ou coupleurs). Un tel procédé permet d'obtenir un éclaircissement de la fibre traitée.

Selon cette deuxième variante, l'agent oxydant peut être ajouté à la composition tinctoriale au moment de l'emploi ou bien il peut encore être mis en oeuvre à partir d'une composition oxydante le comprenant, appliquée simultanément ou séquentiellement à la composition tinctoriale comprenant le colorant mixte. Dans ce dernier cas, l'agent oxydant est présent dans une composition différente de celle comprenant le colorant mixte.

Selon un mode de réalisation particulier, la composition comprenant le colorant mixte est mélangée, de préférence au moment de l'emploi, à une composition contenant, dans un milieu approprié pour la teinture, au moins un agent oxydant, cet agent oxydant étant présent en une quantité suffisante pour obtenir l'éclaircissement souhaité.

Le mélange obtenu est ensuite appliqué sur les fibres kératiniques.

Après un temps de pose suffisant pour obtenir la coloration désirée, habituellement variant de 3 à 50 minutes environ, de préférence 5 à 30 minutes environ, les fibres kératiniques sont de préférence rincées, puis lavées au shampooing, rincées à nouveau puis séchées ou laissées sécher.

Par ailleurs, de manière classique la composition est appliquée et laissée agir à une température allant de 15 à 80°C, de préférence de 15 à 40°C.

La composition oxydante peut également renfermer divers adjuvants utilisés classiquement dans les compositions pour la teinture des fibres kératiniques, notamment humaines, et tels que définis précédemment.

Le pH de la composition oxydante renfermant l'agent oxydant est tel qu'après mélange avec la composition tinctoriale, le pH de la composition résultante appliquée sur les fibres kératiniques (soit en d'autres termes la composition prête à l'emploi) varie de préférence de 7 à 12 environ, et encore plus préférentiellement de 8 à 11. Il peut être ajusté à la valeur désirée au moyen d'agent(s) acidifiant(s) ou alcalinisant(s).

Parmi les agents acidifiants, on peut citer, à titre d'exemples, les acides minéraux ou organiques comme l'acide chlorhydrique, l'acide orthophosphorique, l'acide sulfurique, l'acide acétique.

Pour ce qui concerne les composés alcalinisants, on pourra se reporter à la liste précédemment indiquée.

La composition prête à l'emploi, soit en d'autres termes la composition qui est finalement appliquée sur les fibres kératiniques, peut se présenter sous des formes diverses, telles que sous forme de liquides, de crèmes, de gels ou sous toute autre forme appropriée pour réaliser une teinture des fibres kératiniques, notamment des fibres kératiniques humaines, telles que les cheveux.

L'invention a aussi pour objet un dispositif à plusieurs compartiments dans lequel au moins un premier compartiment comprend une composition tinctoriale comprenant au moins un colorant mixte tel que décrit auparavant, et éventuellement au moins un colorant direct différent du colorant mixte, éventuellement au moins une base d'oxydation, éventuellement au moins un coupleur, et un autre compartiment comprend un agent oxydant.

Il est à noter que le ou les colorants mixtes, éventuellement le colorant direct additionnel, la ou les bases d'oxydation, le ou les coupleurs, peuvent se trouver dans un même compartiment ou dans plusieurs ; un même compartiment pouvant comprendre un seul type de colorant (mixte, direct additionnel, oxydation) ou une combinaison de plusieurs d'entre eux.

Ce dispositif peut être équipé d'un moyen permettant de délivrer sur les fibres à traiter, le mélange souhaité, tel que les dispositifs décrits dans le brevet FR 2 586 913.

Un dernier objet de l'invention est constitué par des colorants mixtes tels qu'ils viennent d'être décrits, ainsi que leurs sels d'addition, à l'exception des composés suivants :

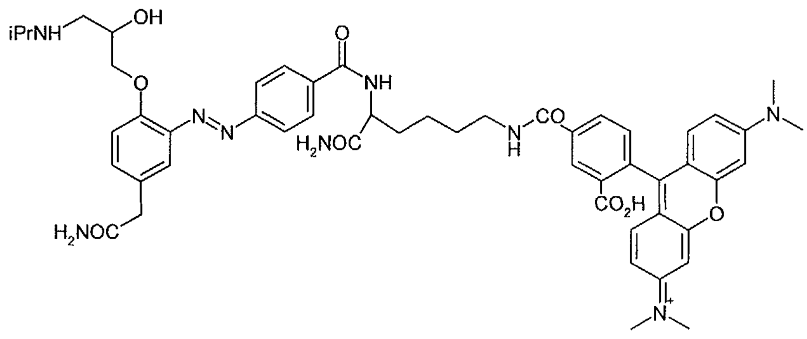

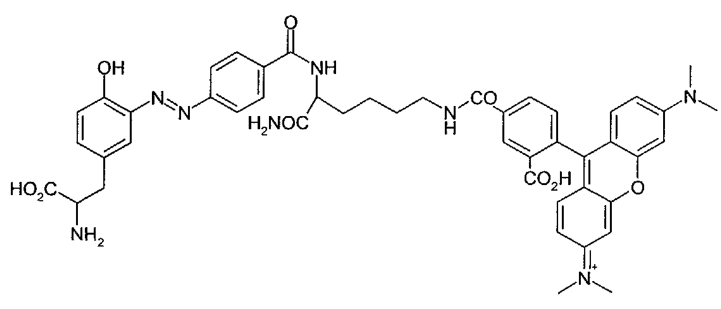

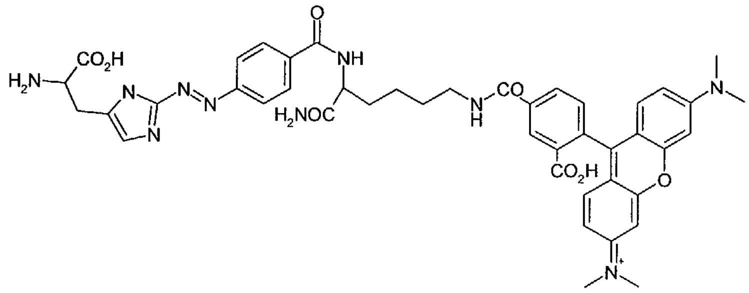

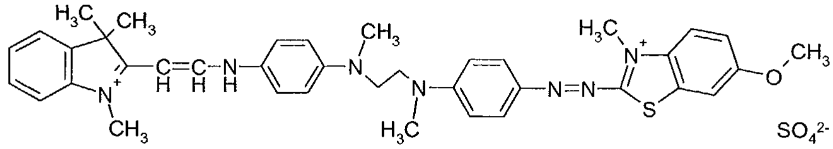

L'invention concerne plus particulièrement des colorants mixtes comprenant deux chromophores. De préférence au moins l'un des deux chromophores porte au moins une charge cationique, avantageusement les deux chromophores portent au moins une charge cationique. Par ailleurs, l'un au moins des chromophores est choisi dans la famille des colorants de type azo, et l'autre est choisi dans la famille des colorants de type diazacarbocyanine ou ses isomères, hémicyanine, nitrés (hétéro)aromatiques. De plus et de manière avantageuse, le bras de liaison correspond à une chaîne alkyle en $C_1$-$C_{20}$, de préférence en $C_1$-$C_{12}$, linéaire ou ramifiée, dont au moins l'un des atomes de carbone peut être remplacé par un hétérocycle comprenant 5 ou 6 chaînons, saturé ou non, comprenant de préférence au moins deux atomes d'azote. Le bras de liaison peut éventuellement porter au moins une charge cationique.

A titre d'exemples de tels colorants mixtes, on peut citer par exemple ceux correspondant aux formules suivantes :

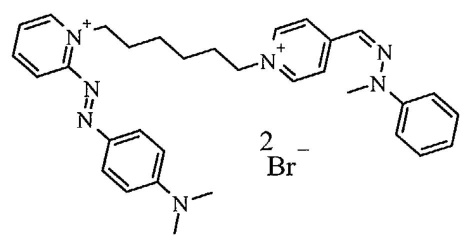

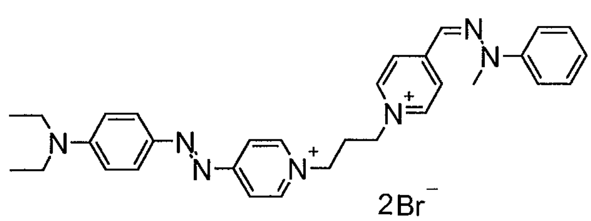

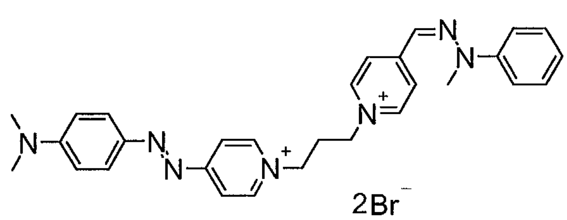

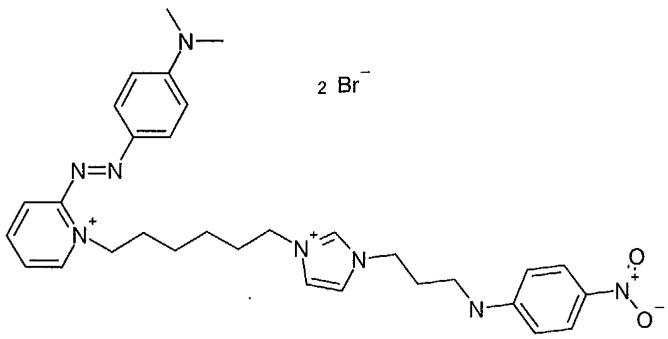

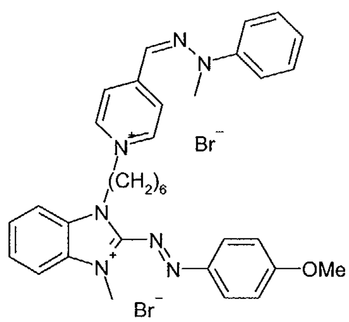

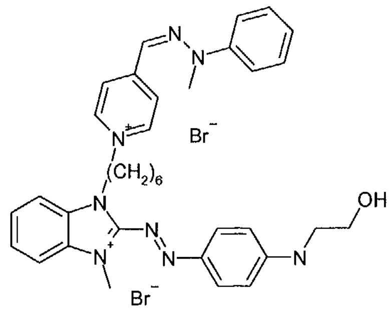

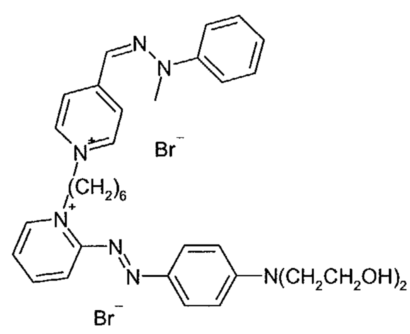

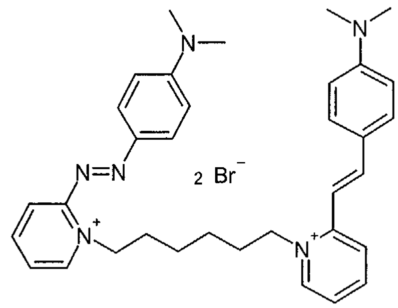

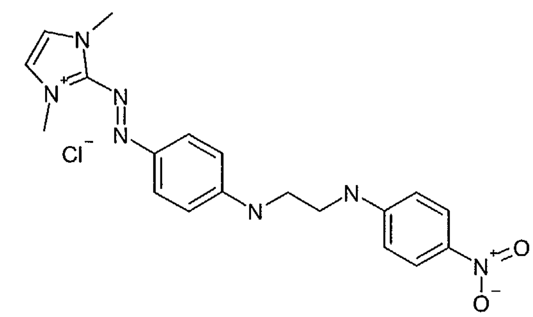

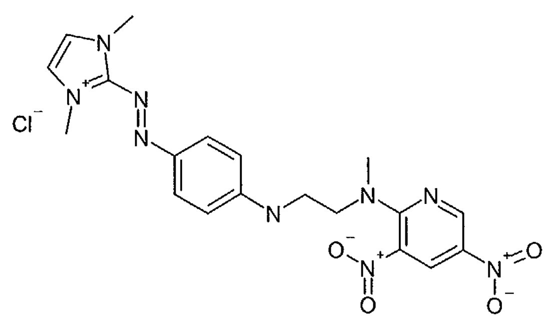

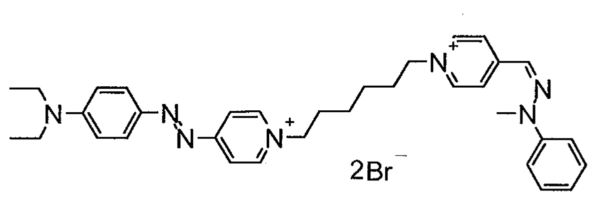

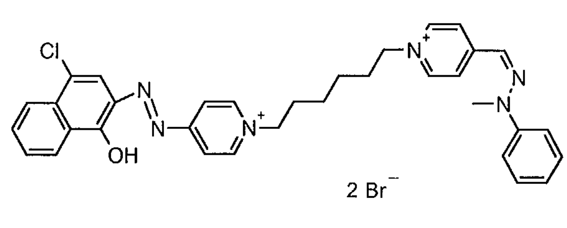

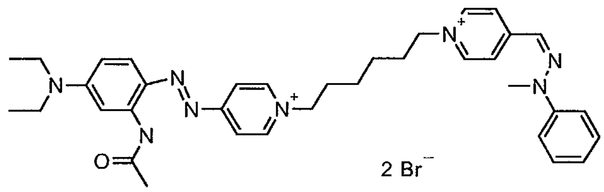

$2\ Br^-$

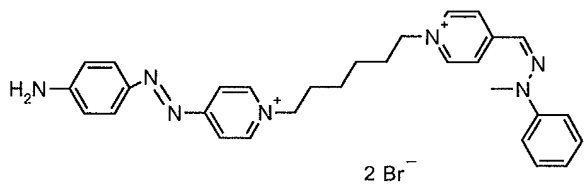

$2\ Br^-$

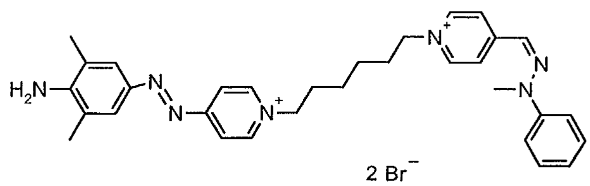

$2\ Br^-$

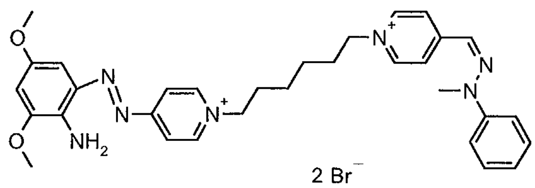

$2\ Br^-$

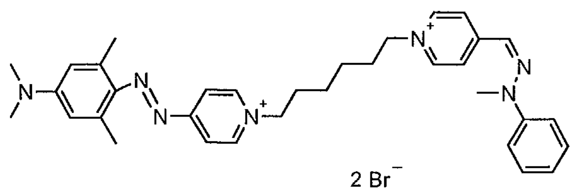

$2\ Br^-$

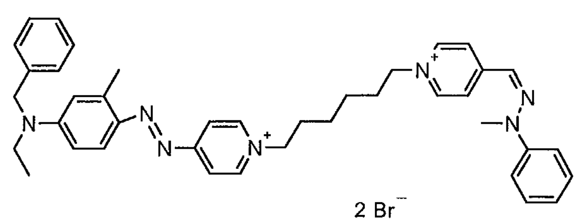

$2\ Br^-$

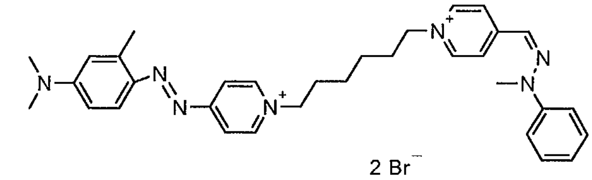

2 $Br^-$

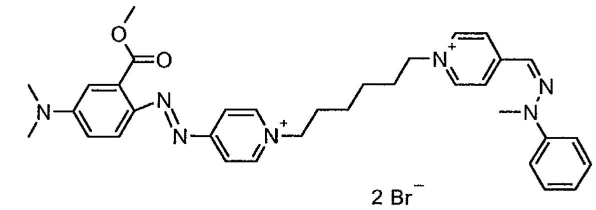

2 $Br^-$

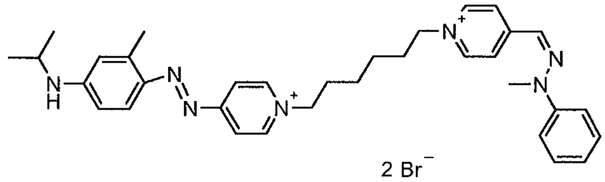

2 $Br^-$

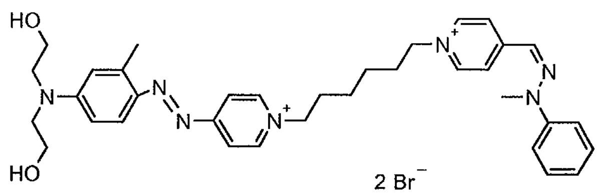

2 $Br^-$

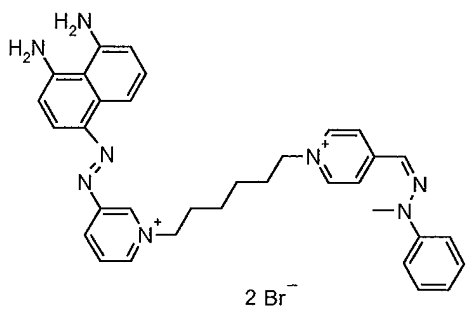

2 $Br^-$

Des exemples concrets mais non limitatifs de la présente invention vont maintenant être présentés

**EXEMPLE 1 : Synthèse du colorant mixte**

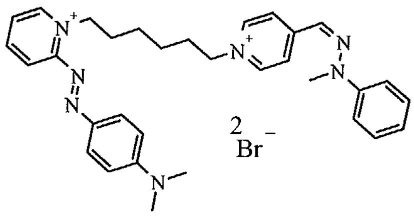

Schéma réactionnel :

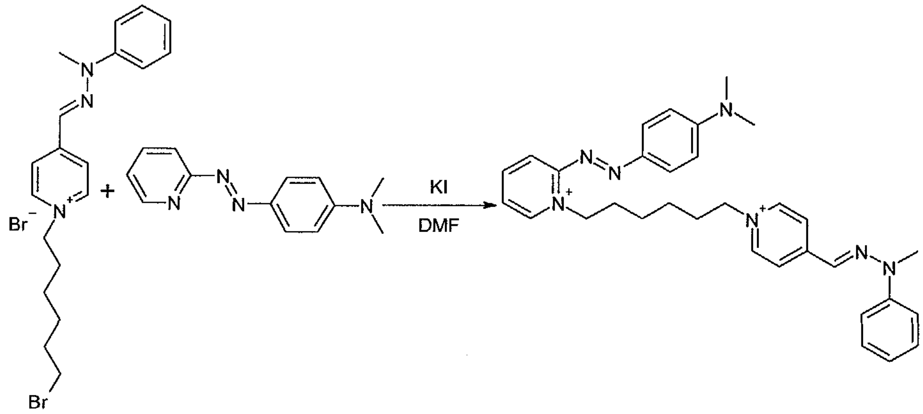


Procédé :

Dans un tricol on introduit 1,06 équivalents du colorant hydrazone (3 g) et environ 100 mg de KI, dans 2 ml de DMF, sous agitation et l'on chauffe à 95°C.
On ajoute ensuite 1 équivalent du colorant azoïque (1,49g) dissout dans 5ml de DMF et on laisse réagir pendant 24 heures.
On récupère le produit par précipitation dans l'acétate d'éthyle (50ml), on filtre et on sèche le produit qui se présente sous la forme d'une poudre noire.
Le produit est purifié par solubilisation dans le dichlorométhane, puis précipité dans un mélange contenant un mélange isopropanol / acétate d'éthyle (1/4) puis filtré.
Le spectre RMN 13C et 1 H sont en accord avec la structure du produit attendu.

**Exemple 2 : Applications en coloration**

**1/ Coloration éclaircissante et non éclaircissante**

Le colorant mixte obtenu dans l'exemple précédent est formulé à $4{,}7 \times 10^{-4}$ mol% dans les compositions de coloration A suivante :

| Composition A | |
|---|---|
| Alkyl ($C_8$/$C_{10}$ 50/50) polyglucoside en solution aqueuse à 60 % tamponnée | 10 g |
| Alcool benzylique | 10 g |
| Polyéthylène glycol 400 à 8 motifs d'oxyde d'éthylène | 12 g |
| Colorant mixte | $4{,}7 \times 10^{-4}$ mol. |
| Ammoniaque à 20.5 % | 13 g |

(suite)

| Composition A | |
|---|---|
| Eau déminéralisée | qsp 100 g |

Au moment de l'emploi, la composition A est mélangée soit avec de l'eau oxygénée 40V (poids pour poids, pH = 3,5) soit avec de l'eau acidifiée (pH = 3,5).
Le pH des compositions tinctoriales après mélange est compris entre 9,5 et 10.
Le mélange est ensuite appliqué sur des mèches de cheveux à 90% blancs naturels (BN) ou permanentés (BP).
Le temps de pose sur mèche est de 20 minutes à température ambiante.
Les mèches subissent ensuite un lavage au shampooing.
On obtient dans les deux cas des mèches colorées.

**2/ Résistance aux shampoings**

Une composition de teinture selon l'invention a été préparée à partir du colorant mixte ($4,7x10^{-4}$ mol%) et du support de teinture A.
Par ailleurs, une composition de teinture comparative a été préparée, dans les mêmes conditions expérimentales, à l'exception du fait que le colorant mixte est remplacé par un mélange équimolaire des deux colorants directs monocationiques monoazoïques constituant ledit colorant mixte.

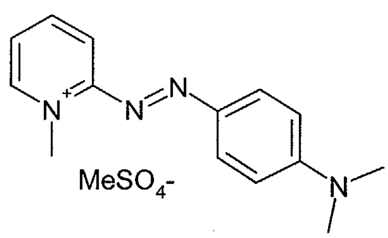

Colorant violet

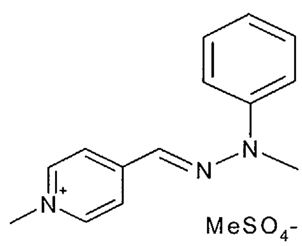

Colorant jaune

Au moment de l'emploi, les compositions ci dessus sont mélangées avec de l'eau oxygénée 40V (poids pour poids, pH = 3,5).
Le pH des compositions tinctoriales après mélange est compris entre 9,5 et 10.
Les mélanges sont ensuite appliqués sur des mèches de cheveux à 90% blancs permanentés (BP).
Le temps de pose sur mèches est de 20 minutes à température ambiante.
Les mèches colorées subissent ensuite six shampooings, avec un séchage intermédiaire entre deux shampooings.
La couleur après les six shampooings est comparée à la couleur initiale de la mèche colorée, visuellement et par mesure colorimétrique.
La résistance aux shampooings est mesurée sur cheveux permanentés colorés selon la formule de $\Delta E$ ci dessous à partir des valeurs de L*a*b* mesurées sur chaque type de mèche avant $L_0^*a_0^*b_0^*$et après les six shampooings $L_1^*a_1^*b_1^*$ (colorimètre CM2002 Minolta, illuminant D65-10° CSI)).

$$\Delta E = \sqrt{(L_1^*-L_0^*)^2 + (a_1^*-a_0^*)^2 + (b_1^*-b_0^*)^2}$$

Les résultats colorimétriques sont regroupés dans le tableau 2.

Tableau 2

| **Composition selon l'invention :** | | | | |
|---|---|---|---|---|
| **Type de cheveux** | **L*** | **a*** | **b*** | **Dégradation** |
| BP / avant shampoings | 22,9 | 15,0 | 3,9 | 2,1 |
| BP / après shampoings | 24,7 | 16,1 | 3,9 | |
| **Composition comparative :** | | | | |
| **Type de cheveux** | **L*** | **a*** | **b*** | **Dégradation** |
| BP / avant shampoings | 24,5 | 18,2 | 6,4 | 4,8 |
| BP / après shampoings | 28,6 | 20,6 | 6,7 | |

Ces résultats montrent que la composition de l'invention présente une bonne résistance aux shampoings.
En outre, sa ténacité est supérieure à celle du mélange des colorants.

**EXEMPLE 2**

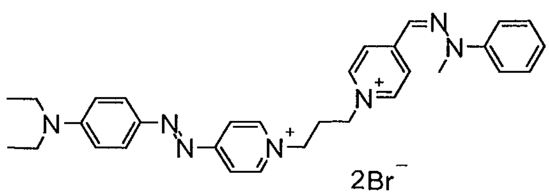

Schéma réactionnel :

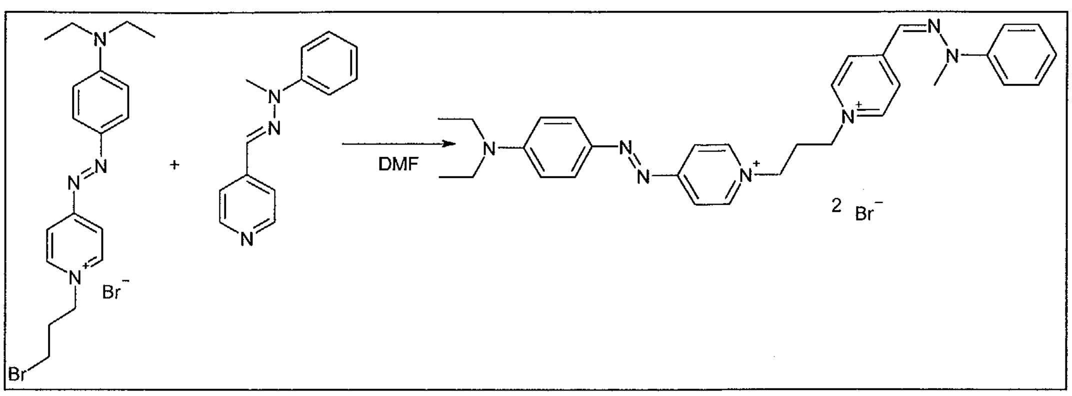


Procédé :

Dans un tricol on introduit le composé azoïque (5 g), 100 ml de DMF et le composé hydrazone (2g). On laisse réagir pendant 4 heures à 100°C. On récupère le produit par précipitation dans le diisopropyle oxyde (250 ml). On filtre et on sèche le produit qui se présente sous la forme d'une poudre noire (2 g).
Les spectres RMN et masse sont en accord avec la structure du produit attendu.
L'utilisation d'une composition tinctoriale du même type que celles décrites dans l'exemple 1 mais comprenant le colorant obtenu dans cet exemple permet d'obtenir des mèches colorées.

EXEMPLE 3

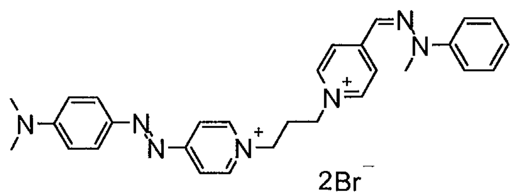

Schéma réactionnel :

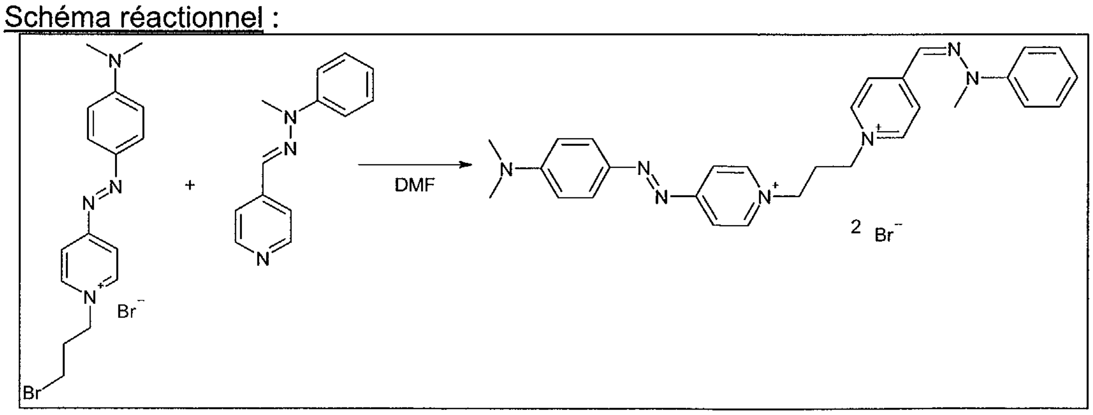


Procédé :

Dans un tricol on introduit le composé hydrazone (98 mg), 10 ml de DMF et le composé azoïque (200 mg). On laisse réagir pendant 4 heures à 100°C. On récupère le produit par précipitation dans l'acétate d'éthyle (100 ml). On filtre et on sèche le produit qui se présente sous la forme d'une poudre noire (200 mg).

Les spectres RMN et masse sont en accord avec la structure du produit attendu.

L'utilisation d'une composition tinctoriale du même type que celles décrites dans l'exemple 1 mais comprenant le colorant obtenu dans cet exemple permet d'obtenir des mèches colorées.

EXEMPLE 4

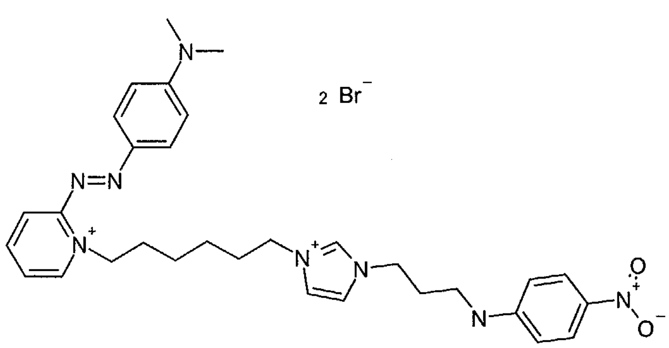

Schéma réactionnel :

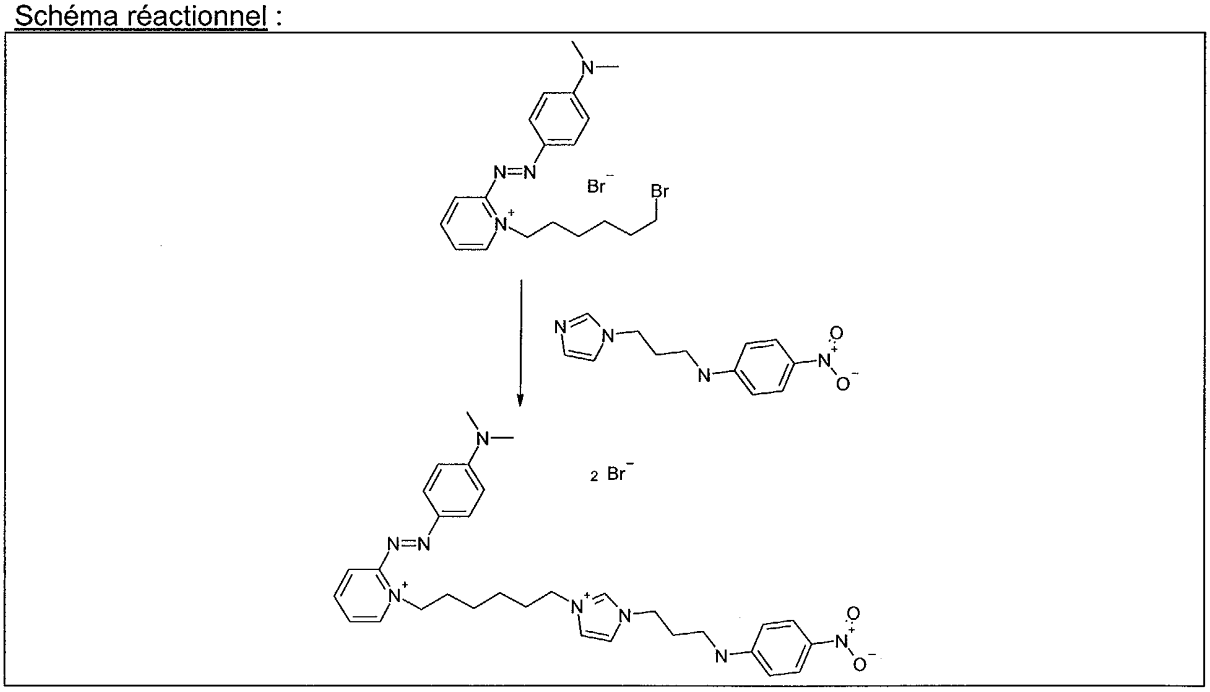

Procédé

Dans un tricol on introduit le composé nitré (3,33 g),100 ml de DMF et le composé azoïque (6,16g). On laisse réagir pendant 4 heures à 100°C. On évapore à sec.

Le produit est repris dans l'oxyde de diisopropyle. On filtre et on sèche sous vide.

On obtient une poudre noire (6 g).

Les spectres RMN et masse sont en accord avec la structure du produit attendu.

L'utilisation d'une composition tinctoriale du même type que celles décrites dans l'exemple 1 mais comprenant le colorant obtenu dans cet exemple permet d'obtenir des mèches colorées.

**EXEMPLE 5**

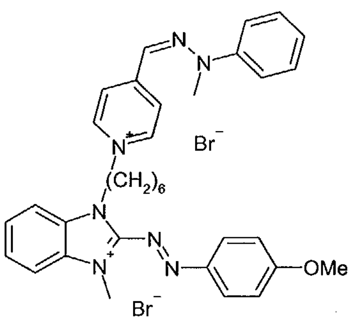

Schéma réactionnel :

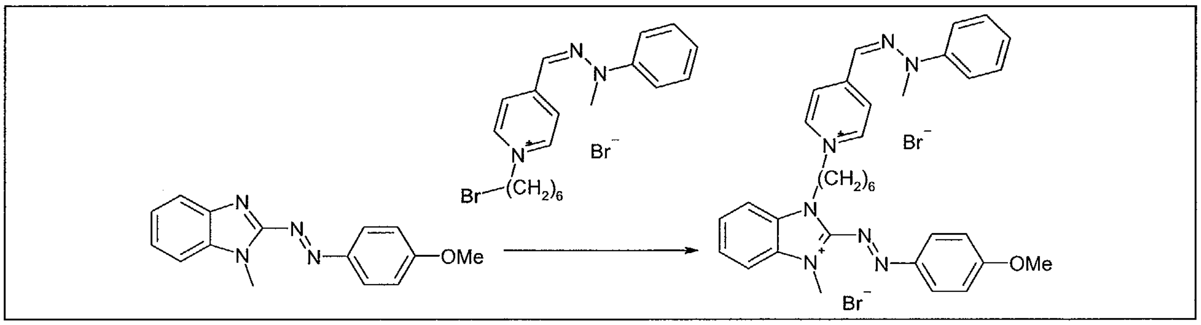

Procédé

Le composé azoïque (1,628g) et le composé hydrazone (2,53 g) sont mis en suspension dans 10 mL d'acétonitrile sec, on ajoute 10 mg d'iodure de potassium et une goutte de DMF.
Le mélange réactionnel est porté à 60°C pendant trois heures puis une fraction d'acétonitrile est évacuée par distillation.
Le mélange réactionnel est à nouveau maintenu à 60°C pendant 14h.
Après refroidissement, le produit (0,5g) est isolé par précipitation dans l'acétone.
Les spectres RMN et masse sont en accord avec la structure du produit attendu.
L'utilisation d'une composition tinctoriale du même type que celles décrites dans l'exemple 1 mais comprenant le colorant obtenu dans cet exemple permet d'obtenir des mèches colorées.

**EXEMPLE 6**

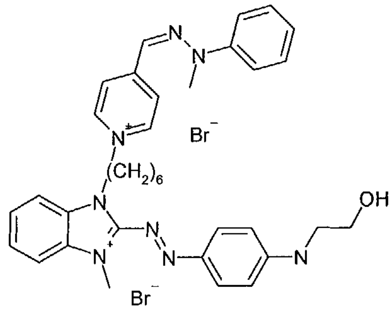

Schéma réactionnel :

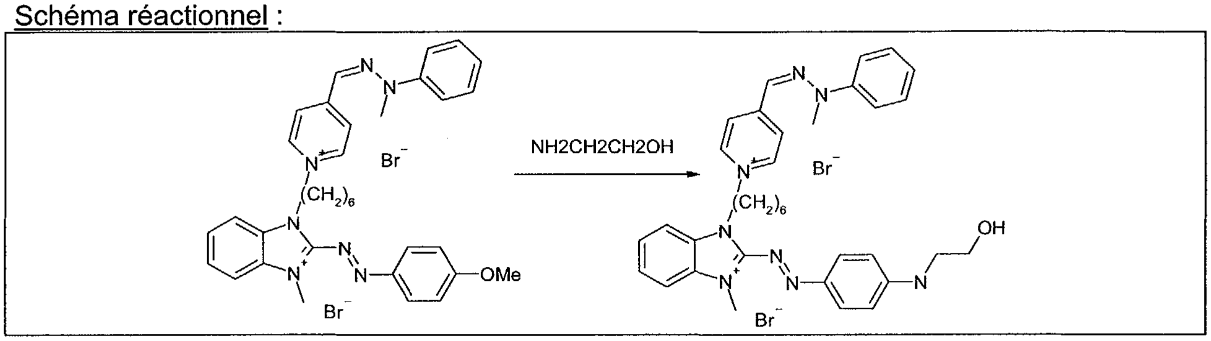

Procédé

200mg de composé obtenu à l'exemple 5 sont dissous dans 2 mL de méthanol, puis on ajoute 1 mL de solution de méthanol contenant 17 mg d'éthanolamine.

Le mélange réactionnel est porté à 60°C pendant une heure, refroidi et dilué dans de l'acétone.

Il se forme un précipité. On recueille après filtration 110 mg de produit conforme.

Les spectres RMN et masse sont en accord avec la structure du produit attendu.

L'utilisation d'une composition tinctoriale du même type que celles décrites dans l'exemple 1 mais comprenant le colorant obtenu dans cet exemple permet d'obtenir des mèches colorées.

**EXEMPLE 7**

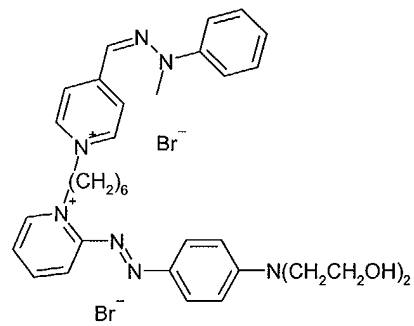

Schéma réactionnel :

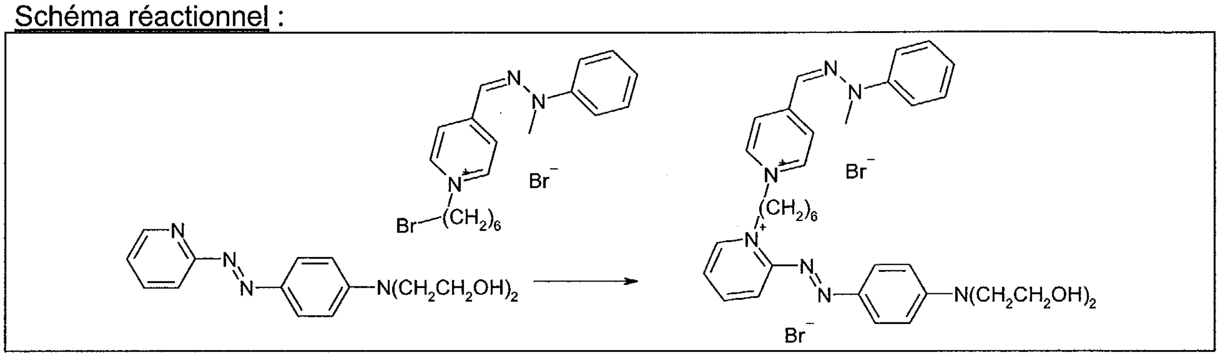

Procédé

Le composé azoïque (0,63g) et le composé hydrazone (1,0 g) sont mis en suspension dans 10 mL DMF sec, on ajoute 224 mg d'iodure de potassium et le mélange réactionnel est porté à 80°C pendant 18 heures.

Le produit (0,441g) est isolé par précipitation dans l'acétate d'éthyle

Les spectres RMN et masse sont en accord avec la structure du produit attendu.

L'utilisation d'une composition tinctoriale du même type que celles décrites dans l'exemple 1 mais comprenant le colorant obtenu dans cet exemple permet d'obtenir des mèches colorées.

**EXEMPLE 8**

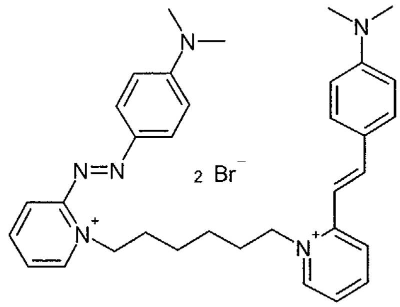

Schéma réactionnel :

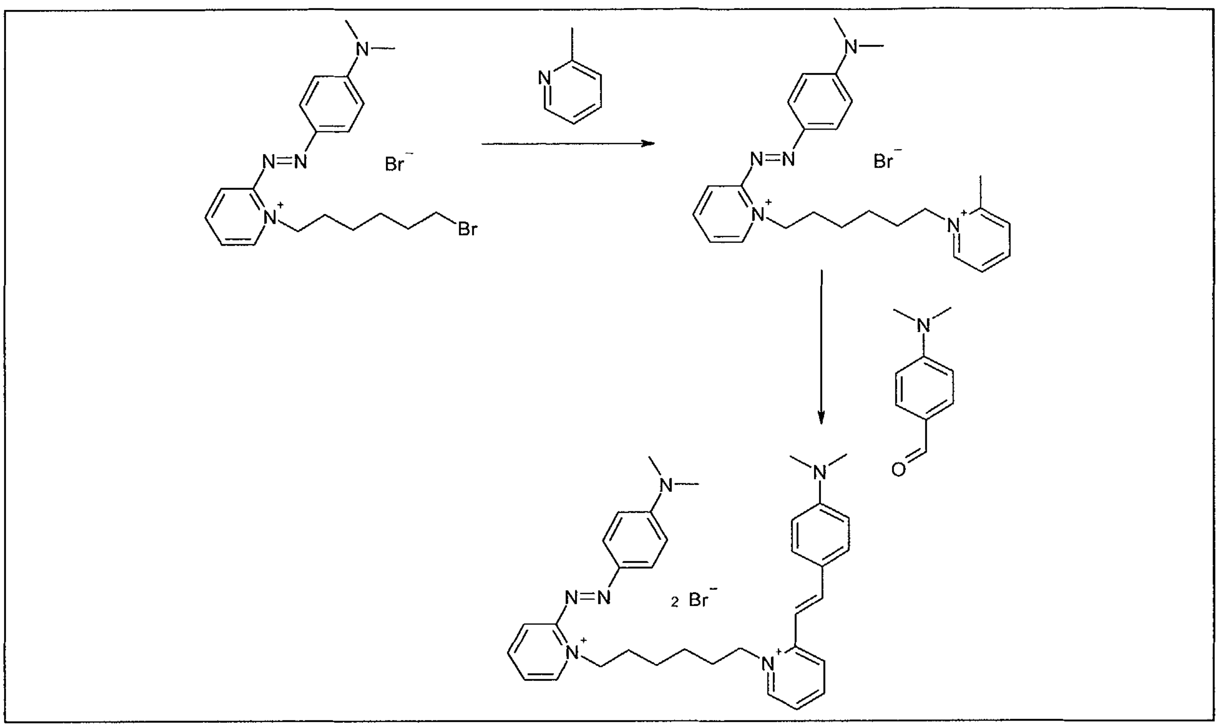

Procédé

Dans un tricol on introduit le composé azoïque (1,0 g), le picoline (2 équivalents molaire) et 30 ml de propane-2-ol.
On laisse réagir avec agitation pendant 24 heures à 85°C puis pendant 3 jours à 92°C. On évapore à sec.
Le produit est repris dans l'eau et lavé avec le dichlorométhane.
On évapore à sec la phase aqueuse. La poudre est reprise dans de l'acétate d'éthyle. On filtre et on sèche sous vide.
On obtient une poudre noire (0,566 g).
Les spectres RMN et masse sont en accord avec la structure de l'intermédiaire.
Dans un tricol on introduit l'intermédiaire obtenu (0,25g), le N,N dimethyl-4-benzaldéhyde (0,112g), la pyrrolidine (0,043ml) et le méthanol (7ml).
On laisse réagir avec agitation pendant 3 jours à température ambiante.

On évapore à sec. On lave avec l'éther de diisopropyle et puis de l'acétate d'éthyle.
On solubilise dans le dichlorométhane (10ml) et on précipite le produit par addition dans une solution d'acétate d'éthyle (90ml) et de propane-2-ol (10ml).
On filtre et on sèche sous vide.
On obtient une poudre noire (0,17g).
Les spectres RMN et masse sont en accord avec la structure du produit.
L'utilisation d'une composition tinctoriale du même type que celles décrites dans l'exemple 1 mais comprenant le colorant obtenu dans cet exemple permet d'obtenir des mèches colorées.

**EXEMPLE 9**

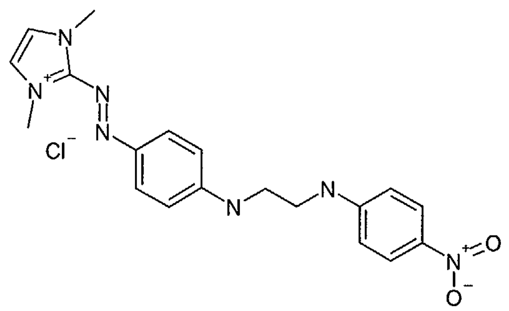

Schéma réactionnel :

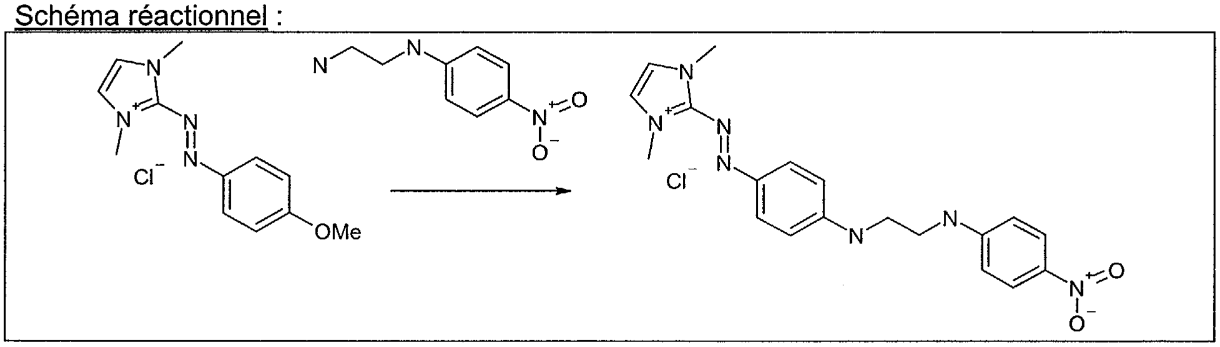

Procédé

Dans un vial on introduit le composé azoïque (1,0 g),7 ml de pentanol et le composé nitré (0,6 g).
On laisse réagir avec agitation pendant 18 heures à 85°C.
On précipite le produit par l'addition de l'acétate d'éthyle. On filtre et on sèche sous vide.
On obtient une poudre noire (1,3 g).
Les spectres RMN et masse sont en accord avec la structure du produit attendu.
L'utilisation d'une composition tinctoriale du même type que celles décrites dans l'exemple 1 mais comprenant le colorant obtenu dans cet exemple permet d'obtenir des mèches colorées.

**EXEMPLE 10**

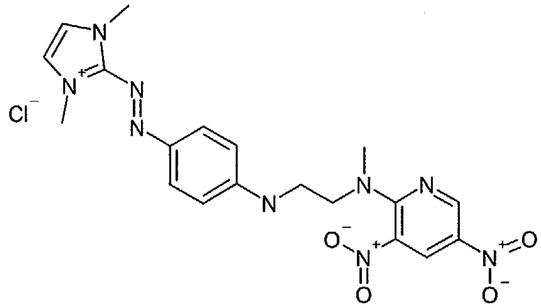

Schéma réactionnel :

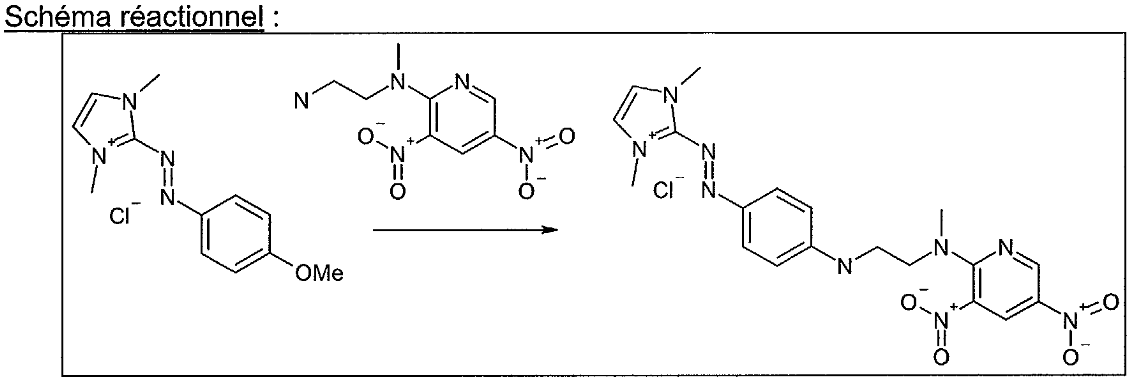

Procédé

Dans un vial on introduit le composé azoïque (1,0 g), 7 ml de pentanol et le composé nitré (0,85 g). On laisse réagir avec agitation pendant 18 heures à 85°C.

On ajoute ensuite le composé nitré (0,42 g) et on laisse réagir avec agitation pendant 3 heures à 90°C. On ajoute enfin le composé nitré (0,85 g).

On laisse réagir avec agitation pendant 2 jours à 90°C.

On précipite le produit par l'addition de l'acétate d'éthyle. On filtre et on lave avec l'acétate d'éthyle et on sèche sous vide.

On obtient une poudre noire (0,84 g).

Les spectres RMN et masse sont en accord avec la structure du produit attendu.

L'utilisation d'une composition tinctoriale du même type que celles décrites dans l'exemple 1 mais comprenant le colorant obtenu dans cet exemple permet d'obtenir des mèches colorées.

**EXEMPLE 11**

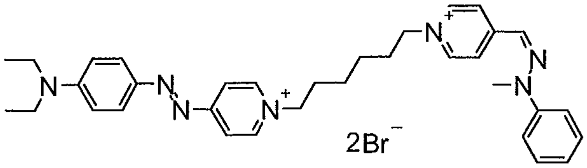

Schéma réactionnel :

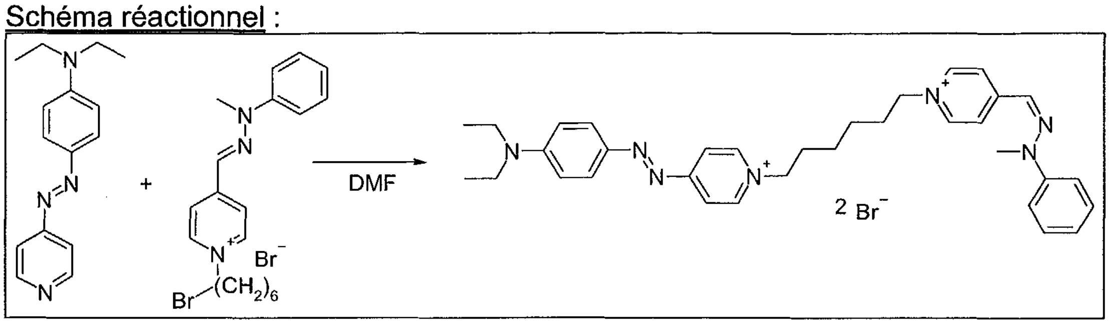


Procédé :

Dans un tricol on introduit le composé azoïque (1 g), KI (100 mg) et 5 ml de diméthylformamide (DMF).
On agite et on chauffe pendant 5 minutes à 90°C.
On ajoute le composé hydrazone (1,79 g) solubilisé dans 5 ml de DMF. On laisse réagir avec agitation pendant 16 heures à 90°C.
On précipite le produit dans l'acétate d'éthyle (250 ml).
On filtre et lave avec l'acétate d'éthyle et on sèche sous vide.
On obtient une poudre noire (2,0g).
Les spectres RMN et masse sont en accord avec la structure du produit attendu.
L'utilisation d'une composition tinctoriale du même type que celles décrites dans l'exemple 1 mais comprenant le colorant obtenu dans cet exemple permet d'obtenir des mèches colorées.
Les exemples 12 à 23 sont obtenus conformément au mode opératoire décrit dans l'exemple 11 en adaptant les quantités et en remplaçant la DMF par la N-méthylpyrrolidone (NMP) ou le DMPU.

**EXEMPLE 12**

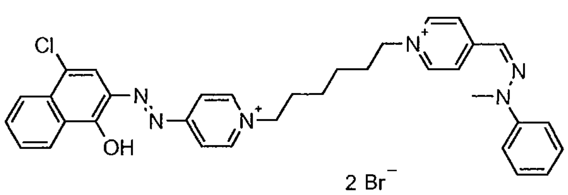

Schéma réactionnel :

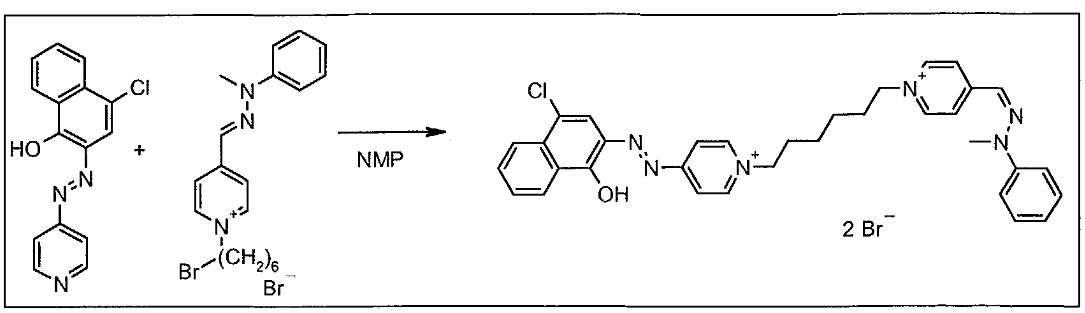


La structure du produit est confirmée par analyse LCMS : pic moléculaire m/z = 289

Longueur d'onde d'absorption maximale : $\lambda_{max}$ : 420 nm ; 566 nm ; 250 nm

**EXEMPLE 13**

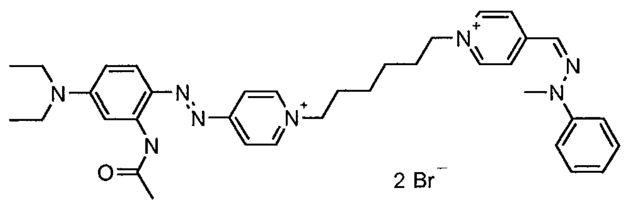

Schéma réactionnel :

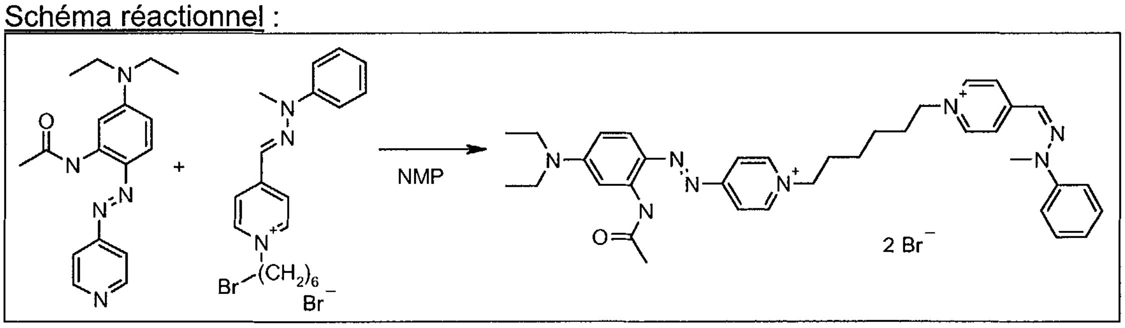


La structure du produit est confirmée par analyse LCMS : pic moléculaire m/z = 303

Longueur d'onde d'absorption maximale : $\lambda_{max}$ : 566 nm ; 420 nm ; 252 nm

**EXEMPLE 14**

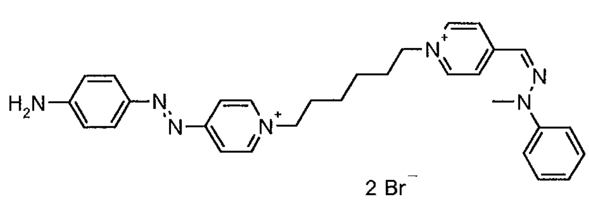

Schéma réactionnel :

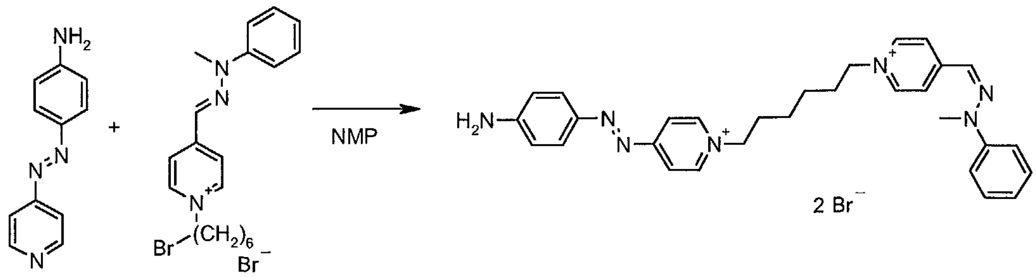

La structure du produit est confirmée par analyse LCMS : pic moléculaire m/z = 247

Longueur d'onde d'absorption maximale : $\lambda_{max}$ : 424 nm ; 518 nm ; 258 nm

**EXEMPLE 15**

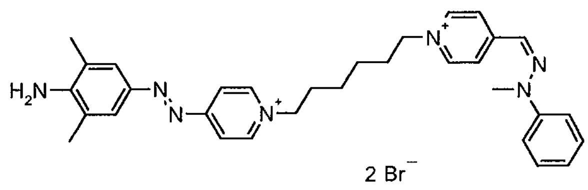

Schéma réactionnel :

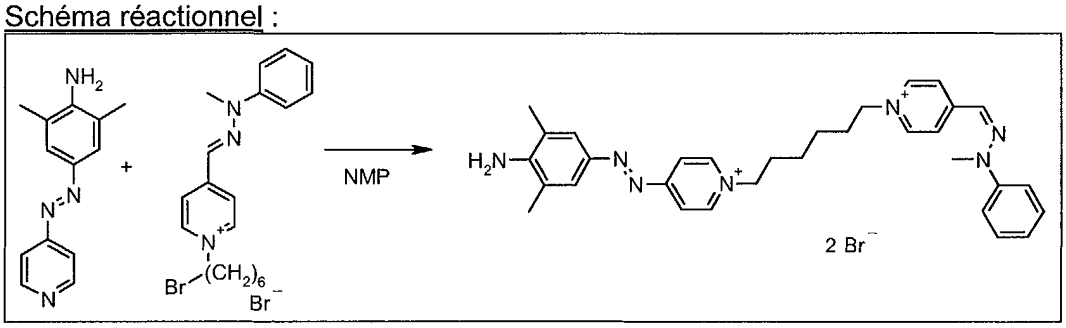


La structure du produit est confirmée par analyse LCMS : pic moléculaire m/z = 261

Longueur d'onde d'absorption maximale : $\lambda_{max}$ : 422 nm ; 538 nm ; 256 nm

**EXEMPLE 16**

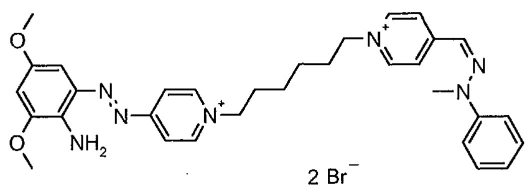

Schéma réactionnel :

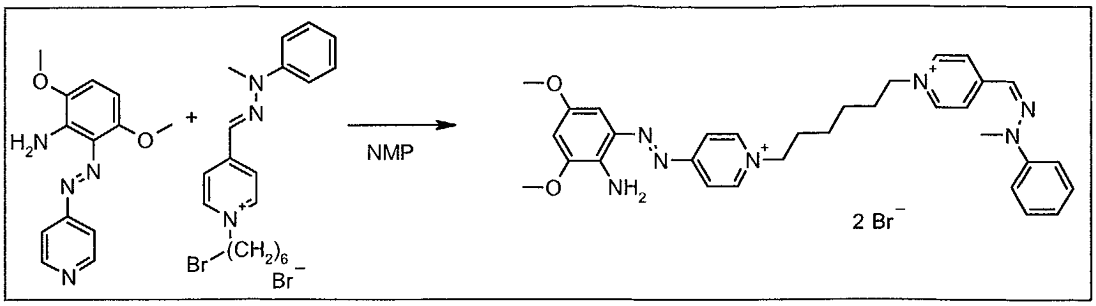


La structure du produit est confirmée par analyse LCMS : pic moléculaire m/z = 277

Longueur d'onde d'absorption maximale : $\lambda_{max}$ : 558nm ; 416 nm ; 254 nm

**EXEMPLE 17**

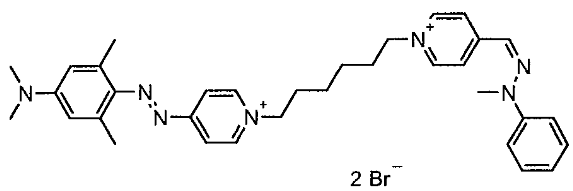

Schéma réactionnel :

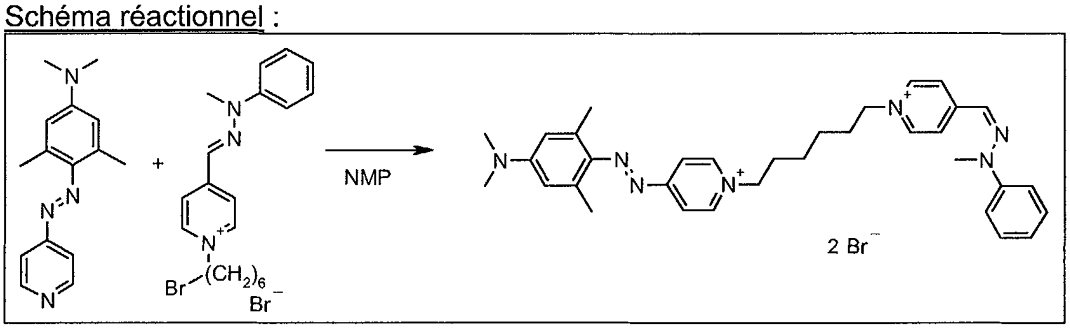


La structure du produit est confirmée par analyse LCMS : pic moléculaire m/z = 275

Longueur d'onde d'absorption maximale : $\lambda_{max}$ : 418 nm ; 552 nm ; 256 nm

**EXEMPLE 18**

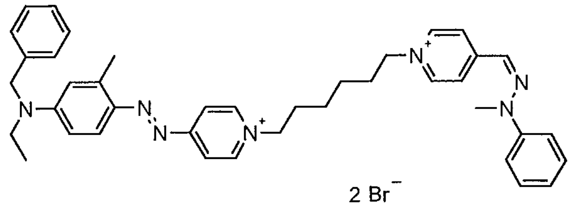

Schéma réactionnel :

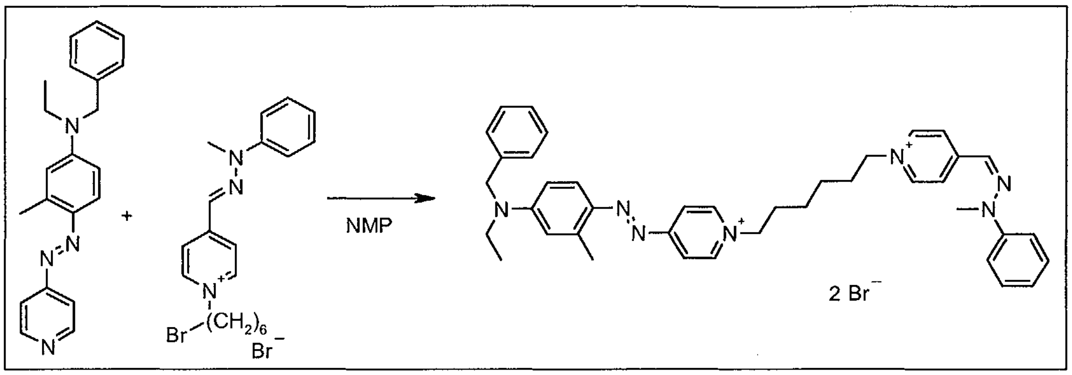


La structure du produit est confirmée par analyse LCMS : pic moléculaire m/z = 313

Longueur d'onde d'absorption maximale : $\lambda_{max}$ : 418 nm ; 568 nm ; 256 nm

**EXEMPLE 19**

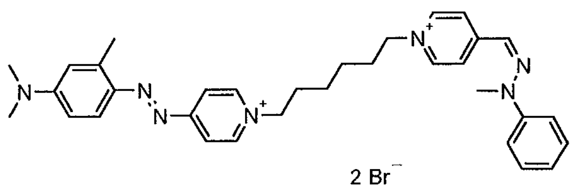

Schéma réactionnel :

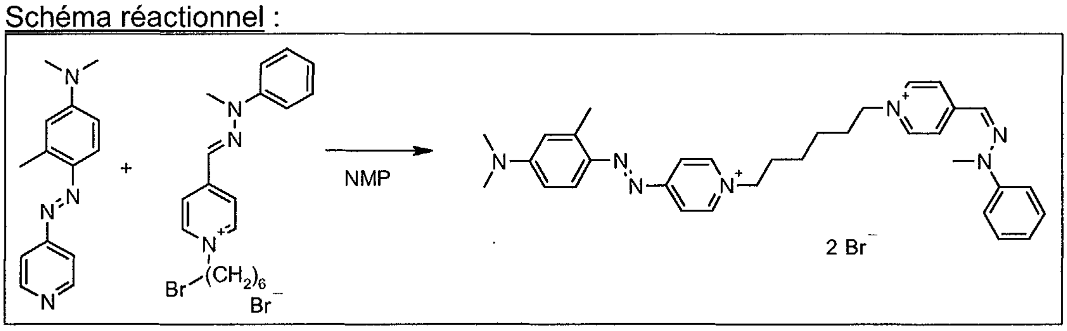


La structure du produit est confirmée par analyse LCMS : pic moléculaire m/z = 268

Longueur d'onde d'absorption maximale : $\lambda_{max}$ : 574 nm ; 420 nm ; 256 nm

**EXEMPLE 20**

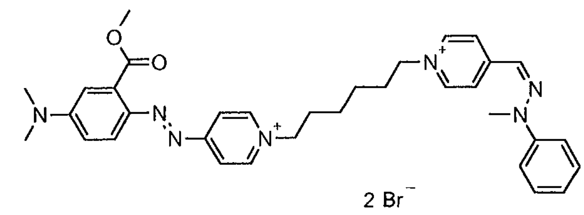

Schéma réactionnel :

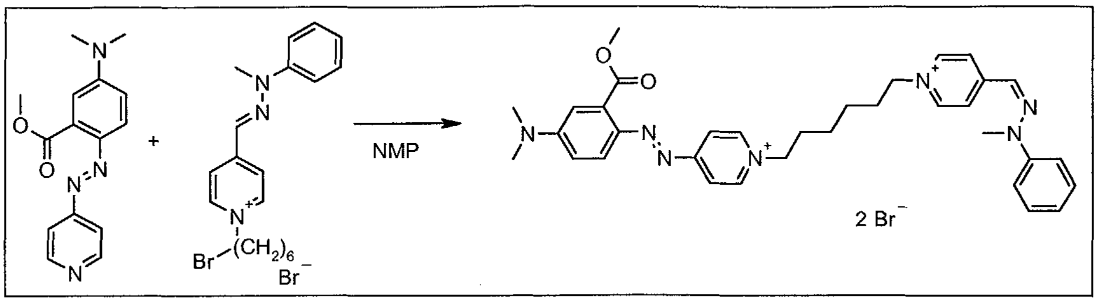


La structure du produit est confirmée par analyse LCMS : pic moléculaire m/z = 290

Longueur d'onde d'absorption maximale : $\lambda_{max}$ : 556 nm ; 420 nm ; 254 nm

**EXEMPLE 21**

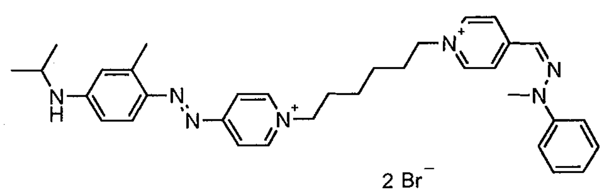

Schéma réactionnel :

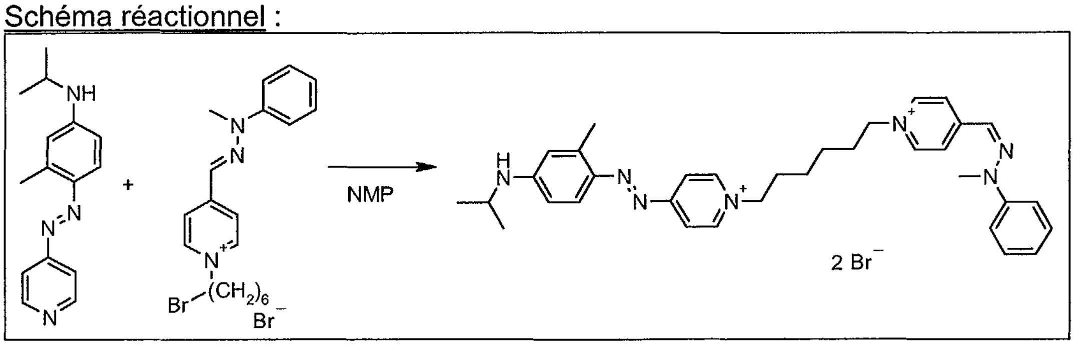

La structure du produit est confirmée par analyse LCMS : pic moléculaire m/z = 275

Longueur d'onde d'absorption maximale : $\lambda_{max}$ : 426 nm ; 562 nm ; 256 nm

**EXEMPLE 22**

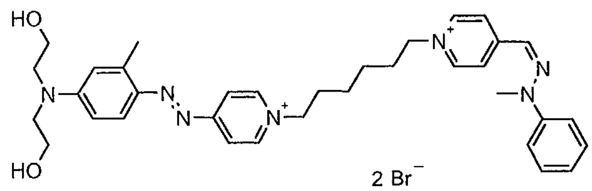

Schéma réactionnel :

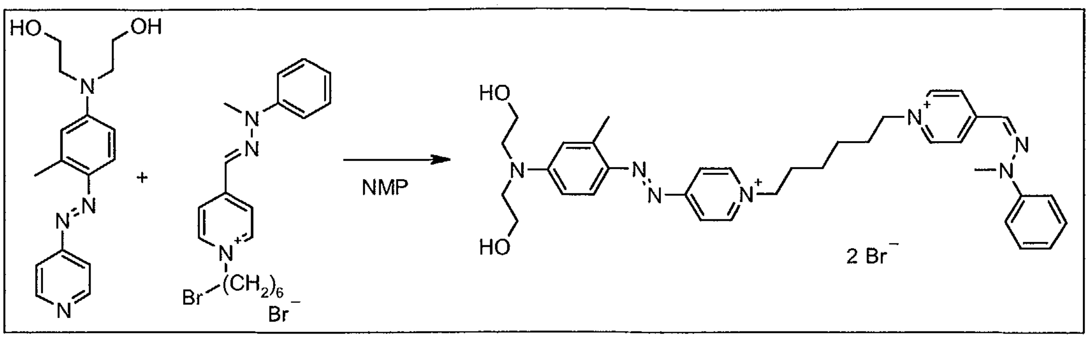


La structure du produit est confirmée par analyse LCMS : pic moléculaire m/z = 298

Longueur d'onde d'absorption maximale : $\lambda_{max}$ : 424 nm ; 572 nm ; 256 nm

**EXEMPLE 23 :**

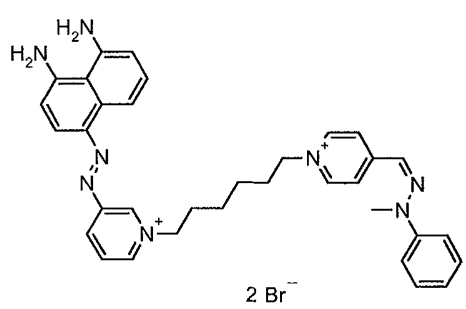

Schéma réactionnel :

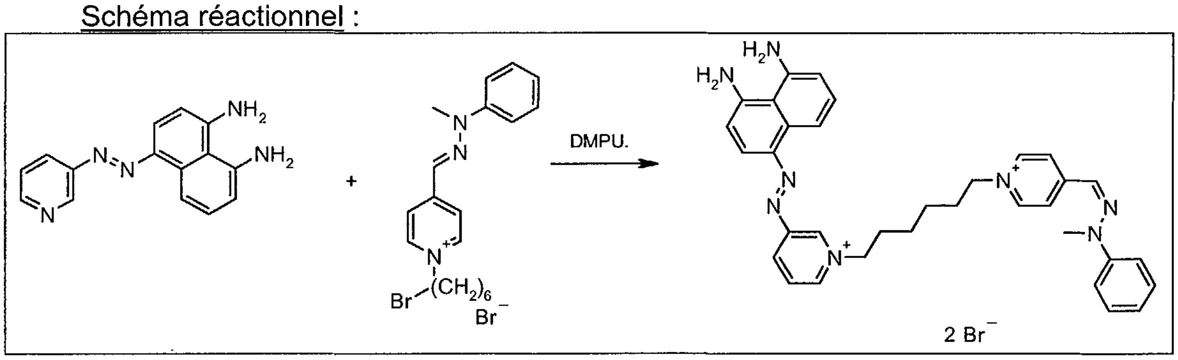


La structure du produit est confirmée par analyse LCMS : pic moléculaire m/z = 279

Longueur d'onde d'absorption maximale : $\lambda_{max}$ : 424 nm ; 590 nm

L'utilisation de compositions tinctoriales du même type que celle décrite dans l'exemple 1 mais comprenant chacun des colorants obtenus dans les exemples 12 à 23 permet d'obtenir des mèches colorées.

## Revendications

1. Composition tinctoriale comprenant, dans un milieu approprié pour la teinture des fibres kératiniques, notamment humaines, au moins un colorant mixte comprenant au moins deux chromophores différents ; au moins l'un des chromophores étant choisi parmi les chromophores de la famille des composés azo ou parmi les chromophores de la famille des tri-(hétéro)aryle méthanes ; les chromophores étant liés entre eux par l'intermédiaire d'au moins un bras de liaison stoppant la délocalisation des électrons de chacun des chromophores ; à l'exclusion de colorants mixtes dont les chromophores sont tous de type azo.

2. Composition selon la revendication précédente, **caractérisée en ce que** le colorant mixte comprend au moins deux chromophores différents dont l'un au moins porte une ou plusieurs charges cationiques.

3. Composition selon l'une quelconque des revendications précédentes, **caractérisée en ce que** les chromophores absorbent dans le domaine visible entre 400 et 800 nm.

4. Composition selon l'une quelconque des revendications précédentes, **caractérisée en ce que** le colorant mixte comprend deux à quatre chromophores, de préférence deux à trois chromophores.

5. Composition selon l'une quelconque des revendications précédentes, **caractérisée en ce que** le colorant mixte comprend deux chromophores.

6. Composition selon l'une des revendications précédentes, **caractérisée en ce que** le ou les chromophores cationiques sont des chromophores comprenant au moins un atome d'azote quaternisé.

7. Composition selon l'une quelconque des revendications précédentes, **caractérisée en ce que** les chromophores de type azo sont choisis parmi les composés comprenant au moins un enchaînement -N=N- dont les deux atomes d'azote ne sont pas simultanément engagés dans un cycle ; sachant qu'il n'est pas exclu que l'un des deux atomes d'azote de l'enchaînement -N=N- soit engagé dans un cycle.

8. Composition selon la revendication précédente, **caractérisée en ce que** les chromophores de type azo sont choisis parmi les des composés azos correspondent à la formule suivante (I), ainsi que ses formes tautomères :

$$A_1 - [N{=}N - (A_3)_y]_x - A_2 \qquad (I)$$

Formule dans laquelle :

- x est un nombre entier compris entre 1 et 3 ;
- y vaut 0 ou 1 ;
- $A_1$, $A_2$, identiques ou non, représentent un radical aromatique en $C_6$-$C_{30}$ ou un radical hétéroaromatique de 5 à 30 chaînons, comprenant au moins un hétéroatome choisi parmi l'azote, l'oxygène et/ou le soufre ; l'un au moins des groupements $A_1$, $A_2$ étant substitué ;
- $A_1$ ou $A_2$ étant lié au bras de liaison du colorant mixte ;
- $A_3$ représente un radical divalent mono- ou poly-aromatique en $C_6$-$C_{30}$, éventuellement substitué, de préférence par un ou plusieurs groupements, identiques ou non, choisis parmi un radical alkyle, linéaire ou ramifié, en $C_1$-$C_6$ ; alcoxy, linéaire ou ramifié, en $C_1$-$C_6$ ; hydroxyle ; amino ; amino substitué par un ou plusieurs radicaux alkyles, identiques ou non, linéaires ou ramifiés, en $C_1$-$C_6$, éventuellement porteurs d'au moins un radical hydroxyle ; nitro ; par un ou plusieurs atomes d'halogène, alkylsulfonamido en $C_1$-$C_{12}$ (alkyl-$SO_2$-NH-), alkylsulfamoyle en $C_1$-$C_{12}$ (alkyl-NH-$SO_2$-), acyloxy dont la partie alkyle est en en $C_1$-$C_{12}$ ; alcoxycarbonyle dont la partie alkyle est en en $C_1$-$C_{12}$ ; carboxyle.

**9.** Composition selon l'une quelconque des revendications précédentes, **caractérisée en ce que** les chromophores de la famille des composés azos correspondent à la formule (I) avec x=1 et y=0, ainsi que ses formes tautomères, dans laquelle :

$A_1$ représente

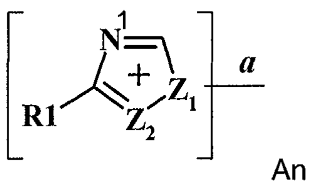

Formule (II)

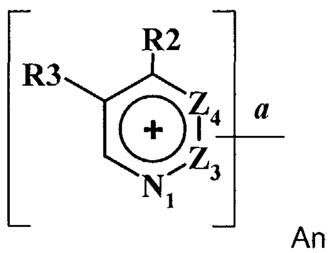

Formule (III)

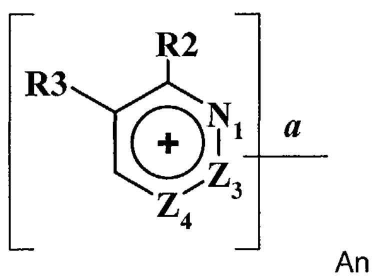

Formule (IV)

la liaison a étant reliée au groupement azoïque soit par l'intermédiaire du cycle ou par l'intermédiaire de $N^1$, $Z_1$, $Z_2$, $Z_3$, $Z_4$, $R_1$, $R_2$, $R_3$ ;

$Z_1$ représente un atome d'oxygène, de soufre, un radical $NR_4$ ou un radical $CR_5$,

$Z_2$ représente un atome d'azote ou un radical $CR_6$,

$Z_3$ représente un atome d'azote ou un radical $CR_7$,

$Z_4$ représente un atome d'azote ou un radical $CR_8$,

$R_1$ et $R_6$ peuvent former ensemble un cycle aromatique,

An représente un anion cosmétiquement acceptable,

$A_2$ représente un groupement aromatique en $C_6$-$C_{30}$ ou hétéroaromatique comprenant de 5 à 30 chaînons, éventuellement substitué, éventuellement cationique, lié au groupement azoïque soit par l'intermédiaire dudit cycle, soit par l'intermédiaire de l'un de ses substituants, et de préférence, $A_2$ représente un groupement aromatique carboné ou pyridinique de formule :

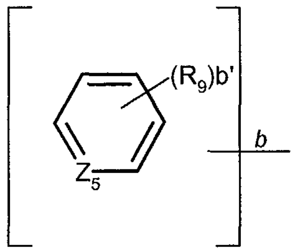

Formule (V) Formules dans lesquelles :

La liaison b étant reliée au groupement azoïque par l'intermédiaire du cycle ;
$Z_5$ représente un atome d'azote ou un radical $CR_{10}$ ;
$R_1$, $R_2$, $R_3$, $R_5$, $R_6$, $R_7$, $R_8$, $R_9$, et $R_{10}$, identiques ou différents, représentent un atome d'hydrogène ; un radical alkyle, linéaire ou ramifié, en $C_1$-$C_8$, éventuellement substitué ; un radical (hétéro)aryle comprenant 5 ou 6 chaînons, éventuellement substitué ; un atome d'halogène ; un groupement hydroxyle ; un radical amino ; un radical amino substitué par un ou plusieurs radicaux alkyles, identiques ou non, linéaires ou ramifiés en $C_1$-$C_6$, éventuellement porteurs d'au moins un groupement hydroxyle ; un groupement nitro ; un groupement cyano ; alkylsulfonamido en $C_1$-$C_{12}$ (alkyl-$SO_2$-NH-) ; alkylsulfamoyle en $C_1$-$C_{12}$ (alkyl-NH-$SO_2$-) ; acyloxy dont la partie alkyle est en en $C_1$-$C_{12}$ ; alcoxycarbonyle dont la partie alkyle est en en $C_1$-$C_{12}$ ; un groupement carboxy ;
$R_4$ représente un radical alkyle, linéaire ou ramifié, en $C_1$-$C_8$, éventuellement substitué ;
$R_1$ avec $R_5$, et $R_6$ avec $R_7$ peuvent former un cycle aromatique ;
Le coefficient b' est égal à 4 ;
An représente un anion cosmétiquement acceptable de nature organique ou minérale.

**10.** Composition selon l'une des revendications précédentes, **caractérisée en ce que** les chromophores de type tri-(hétéro)aryle méthanes sont choisis parmi les des composés de formules (VI) à (VIII) suivantes :

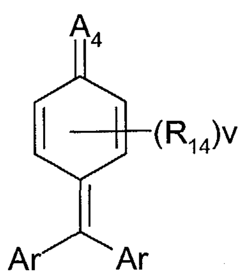

(VI)

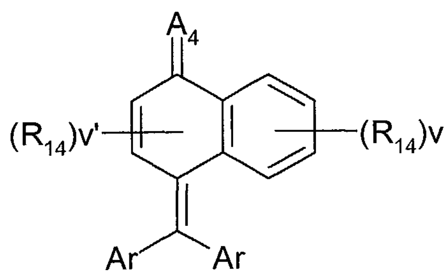

(VII)

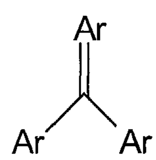

(VIII)

dans lesquelles :

- Ar, identiques ou non, représentent un radical aryle, tel que phényle ou naphtyle, éventuellement substitué ; un hétérocycle, éventuellement substitué ;
- $A_4$ représente O, N-$R_{15}$, $N^+(R_{16})_2$ dans lesquelles $R_{15}$, $R_{16}$, indépendamment les uns des autres, représentent un atome d'hydrogène, un radical alkyle, linéaire ou ramifié, en $C_1$-$C_8$ éventuellement substitué, de préférence par au moins un hydroxyle ; un groupement amido (-$CONH_2$), un groupement alcoxy en $C_1$-$C_4$ ; un radical amino ; un radical amino substitué par un ou plusieurs radicaux alkyles, identiques ou non, linéaires ou ramifiés, en $C_1$-$C_4$, éventuellement porteurs d'un groupement hydroxyle.
- $R_{14}$, identiques ou non, représentent un atome d'hydrogène ; un atome d'halogène, de préférence le chlore, le fluor ; un groupe sulfonylamino ; un hydroxyle ; un radical alkyle, linéaire ou ramifié, en $C_1$-$C_8$ éventuellement substitué ; un radical alcoxy, linéaire ou ramifié, en $C_1$-$C_8$, éventuellement substitué ; un radical alkylthio, linéaire ou ramifié, en $C_1$-$C_8$ éventuellement substitué ; un radical amino ; un radical amino substitué par un ou plusieurs radicaux alkyle, identiques ou non, linéaires ou ramifiés, en $C_1$-$C_8$, éventuellement porteurs d'au moins un groupement hydroxyle ; un radical hétérocyclique ; un groupement nitro ; un groupement cyano ; un radical aryle, de préférence en $C_6$, éventuellement substitué ; un groupement acyle ; un radical alcoxycarbonyle, linéaire ou ramifié, en $C_1$-$C_8$ ; un groupement carboxamido ; -$CO_2H$ ; -$SO_3H$ ; -$PO_3H_2$ ; -$PO_4H_2$ ;
- v est égal à 4 ; v' est égal à 2.

**11.** Composition selon l'une quelconque des revendications précédentes, **caractérisée en ce que** le chromophore peut être choisi parmi les chromophores de type méthines, carbonyles, azines cycliques, composés nitrés (hétéro) aromatiques.

**12.** Composition selon la revendication précédente, **caractérisée en ce que** les chromophores de type méthines, sont choisis parmi les composés comprenant au moins un enchaînement choisi parmi >C=C< et -N=C< dont les deux atomes ne sont pas simultanément engagés dans un cycle ; sachant qu'il n'est pas exclu que l'un des atomes d'azote ou de carbone des enchaînements peut être engagé dans un cycle.

**13.** Composition selon l'une quelconque des revendications 11 ou 12, **caractérisée en ce que** les chromophores de type méthines sont des radicaux issus de chromophores de la famille des méthine, azométhine, mono- et di-arylméthane, indamines, indophénols, indoanilines, carbocyanines, azacabocyanines et leurs isomères, diazacarbocyanines et leurs isomères, tétraazacarocyanines, hémicyanines, ainsi que, le cas échéant, leurs isomères.

**14.** Composition selon l'une quelconque des revendications 11 à 13, **caractérisée en ce que** les chromophores de type méthine sont des radicaux issus de chromophores de formule suivante (IX) ainsi que ses formes tautomères :

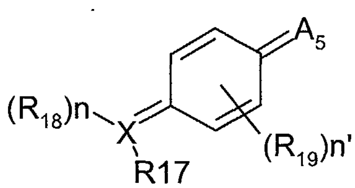

Formule (IX) dans laquelle :

- $R_{17}$, $R_{18}$, identiques ou non, représentent un atome d'hydrogène ; un radical aryle en $C_6$-$C_{30}$ un radical alkyl ($C_1$-$C_8$)aryle, la partie aryle étant éventuellement substituée par un ou plusieurs groupements, identiques ou non, choisis de préférence parmi les groupements hydroxyle, alcoxy, linéaire ou ramifié, substitué ou non, en $C_1$-$C_4$, amino, amino substitué par un ou plusieurs radicaux alkyles, identiques ou non, linéaires ou ramifiés, en $C_1$-$C_4$ éventuellement porteurs d'au moins un groupement hydroxyle, les atomes d'halogène, de préférence le chlore ; un radical hétérocyclique, choisi notamment parmi les hétérocycles thiophène, furane, pipéronyle, indole, indoline, pyridine, carbazole, déhydroquinoléïne, chromone ;
- $R_{17}$, $R_{18}$, ne peuvent en outre représenter simultanément ni un radical aromatique, ni un radical hétéroaromatique ;
- $R_{19}$, identiques ou différents, représentent un atome d'hydrogène ; un radical alkyle, linéaire ou ramifié, en $C_1$-$C_8$ éventuellement substitué ; un radical aryle en $C_6$-$C_{30}$, éventuellement substitué ; un radical amino ; un radical amino substitué par ou plusieurs radicaux alkyle, linéaires ou ramifiés, en $C_1$-$C_4$, éventuellement porteurs d'au moins un groupement hydroxyle ; un groupement hydroxyle ; un radical alcoxy, linéaire ou ramifié, en $C_1$-$C_8$, éventuellement porteur d'au moins un groupement hydroxyle, alcoxy en $C_1$-$C_4$ ; un atome d'halogène et de préférence le chlore ;
- X représente un atome d'azote, un atome de carbone ;
- le coefficient n représente 0 lorsque X est un atome d'azote, et 1 lorsque X est un atome de carbone ;
- le coefficient n' est égal à 4 ;
- $A_5$ représente un groupement amino ; un groupement amino substitué par un ou plusieurs radicaux alkyles en $C_1$-$C_8$, identiques ou non, linéaires ou ramifiés, éventuellement porteurs d'au moins un groupement hydroxyle, un groupement ammonium $N^+(R_{20})_2$ $R_{20}$, identiques ou non, représentent un radical alkyle en $C_1$-$C_8$ éventuellement substitué ; un radical aryle en $C_6$ éventuellement substitué, de préférence par un ou plusieurs hydroxyle, atomes d'halogène, de préférence le chlore, le fluor, groupes nitro, groupes cyano, alcoxy, linéaires ou ramifiés, en $C_1$-$C_4$, monohydroxy alcoxy en $C_1$-$C_4$, linéaires ou ramifiés, polyhydroxyalcoxy, en $C_2$-$C_4$, linéaires ou ramifiés, amino substitués ou non par un ou plusieurs radicaux alkyle, hydroxyalkyle, identiques ou différents, en $C_1$-$C_4$, linéaires ou ramifiés.

Ainsi que ceux issus de la formule suivante (X) :

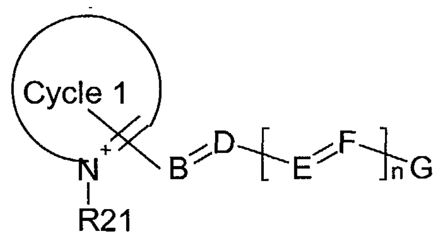

et le cas échéant leurs formes tautomères ;
formule dans laquelle

B, D, E, et F, identiques ou différents, représentant un atome d'azote ou un groupe C-$R_{22}$,
$R_{22}$, identiques ou non, représentent un atome d'hydrogène, un radical alkyle en $C_1$-$C_8$ éventuellement substitué, de préférence par au moins un hydroxyle ; un radical alcoxy, linéaire ou ramifié, en $C_1$-$C_4$ ; un radical amino ; un radical amino substitué par un ou plusieurs radicaux alkyle, identiques ou non, linéaires ou ramifiés, en $C_1$-$C_4$, éventuellement porteurs d'au moins un groupe hydroxyle ; un radical aryle en $C_6$ éventuellement substitué, un radical hétéroaryle comprenant de 5 à 12 chaînons, éventuellement substitué ;
n = 0 ou 1 ;
G représentant un Cycle 4, ou les restes :

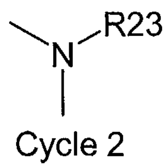

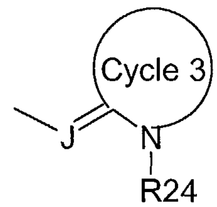

formules dans lesquelles :

$R_{21}$ et $R_{24}$ représentent indépendamment l'un de l'autre, un radical alkyle, linéaire ou ramifié, en $C_1$-$C_8$ éventuellement substitué ; un radical benzyle éventuellement substitué ;
$R_{23}$ représente un atome d'hydrogène, un radical alkyle en $C_1$-$C_8$ éventuellement substitué, de préférence par au moins un hydroxyle, un radical alcoxy, linéaire ou ramifié, en $C_1$-$C_4$, un radical amino, un radical amino substitué par un ou plusieurs radicaux alkyle, identiques ou non, linéaires ou ramifiés, en $C_1$-$C_4$, éventuellement porteurs d'au moins un groupe hydroxyle ; un radical aryle en $C_6$ éventuellement substitué ; un radical hétéroaryle en $C_2$-$C_{12}$ éventuellement substitué ;
**J** représente un atome d'azote ou un groupe C-$R_{25}$ ; avec $R_{25}$ ayant la même signification que $R_{22}$ ;
**Cycle 1** étant choisi parmi les radicaux hétéroaromatiques à 5 à 12 chaînons, portant au moins une charge cationique sur un atome d'azote et comprenant éventuellement au moins un autre hétéroatome choisi parmi l'azote, l'oxygène et/ou le soufre ; lesdits radicaux étant éventuellement substitués par au moins un radical alkyle, linéaire ou ramifié, en $C_1$-$C_8$ substitué ou non ; au moins un radical alcoxy, linéaire ou ramifié, en $C_1$-$C_8$ substitué ou non ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical aromatique en $C_5$-$C_6$ ; au moins un groupement hydroxyle ; alcoxycarbonyle ; nitro ; cyano ; alkylsulfonamido en $C_1$-$C_{12}$ (alkyl-$SO_2$-NH-) ; alkylsulfamoyle en $C_1$-$C_{12}$ (alkyl-NH-$SO_2$-) ;
**Cycle 2** étant choisi parmi les radicaux aromatiques en $C_6$-$C_{12}$ ; les radicaux hétéroaromatiques à 5 à 12 chaînons, comprenant au moins un hétéroatome choisi parmi l'azote, l'oxygène et/ou le soufre ; lesdits radicaux étant éventuellement substitués par au moins un radical alkyle, linéaire ou ramifié, en $C_1$-$C_8$ ; au moins un radical alcoxy, linéaire ou ramifié, en $C_1$-$C_8$ ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical (hétéro)aromatique comprenant de préférence 5 à 6 chaînons ; au moins un groupement hydroxyle ;
**Cycle 3** étant choisi parmi les radicaux hétéroaromatiques à 5 ou 6 chaînons comprenant au moins un hétéroatome choisi parmi l'azote, l'oxygène et/ou le soufre ; lesdits radicaux étant éventuellement substitués par

au moins un radical alkyle, linéaire ou ramifié, en $C_1$-$C_8$ ; au moins un radical alcoxy, linéaire ou ramifié, en $C_1$-$C_8$ ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical aromatique en $C_5$-$C_6$ ; au moins un groupement hydroxyle ; alcoxycarbonyle ; nitro ; cyano ; alkylsulfonamido en $C_1$-$C_{12}$ (alkyl-$SO_2$-NH-) ; alkylsulfamoyle en $C_1$-$C_{12}$ (alkyl-NH-$SO_2$-) ;

**Cycle 4** étant choisi parmi les radicaux aromatiques en $C_6$-$C_{12}$ ; les radicaux hétéroaromatiques à 5 à 12 chaînons, comprenant au moins un hétéroatome choisi parmi l'azote, l'oxygène et/ou le soufre ; lesdits radicaux étant éventuellement substitués par au moins un radical alkyle, linéaire ou ramifié, en $C_1$-$C_8$ ; au moins un radical alcoxy, linéaire ou ramifié, en $C_1$-$C_8$ ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical (hétéro)aromatique comprenant de préférence 5 à 6 chaînons; au moins un groupement hydroxyle ;

à la condition que lorsque n vaut 1 et G représente un cycle, alors B, D, E et F ne peuvent représenter simultanément un atome d'azote ; et que lorsque n vaut 0 et

G représente un cycle, alors B et D ne représentent pas simultanément un atome d'azote.

**15.** Composition selon la revendications 10, **caractérisée en ce que** le chromophore de la famille des carbonyles est un radical issu de colorants de la famille les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

**16.** Composition selon l'une quelconque des revendications 10 ou 15, **caractérisée en ce que** le chromophore de la famille des carbonyles est un radical issu de colorants de la formule suivante (XI) :

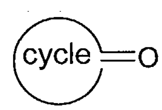

Formule dans laquelle le cycle représente un cycle à 5 ou 6 chaînons, dont éventuellement au moins l'un d'entre eux est remplacé par un hétéroatome choisi parmi l'oxygène, l'azote, le soufre, ou par une fonction carbonyle additionnelle ; ledit cycle étant éventuellement substitué par un radical ou plusieurs radicaux choisis parmi les radicaux alkyle, linéaire ou ramifié, en $C_1$-$C_8$, éventuellement substitués ; par un ou plusieurs radicaux hydroxyle ; les atomes d'halogène comme de préférence le chlore ; les groupements nitro, cyano, amino, alkylamino ; ledit cycle étant éventuellement condensé avec un ou plusieurs cycles aromatiques en $C_6$, ce ou ces cycles pouvant eux-mêmes être condensés à un ou plusieurs cycles aromatiques dont éventuellement au moins l'un des atomes de carbone est remplacé par un ou plusieurs hétéroatomes choisis parmi l'oxygène, l'azote ou le soufre.

**17.** Composition selon la revendication 10, **caractérisée en ce que** le chromophore de la famille des azines cycliques est choisi parmi les radicaux issus de colorants de type azine, xanthène, thioxanthène, fluorindine, acridine, (di) oxazine, (di)thiazine, pyronine.

**18.** Composition selon l'une quelconque des revendications 10 ou 17, **caractérisée en ce que** le chromophore de la famille des azines cycliques est un radical issu de colorants formule suivante (XII) ainsi que ses formes tautomères :

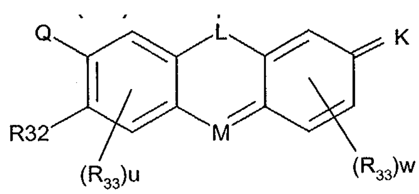

formule dans laquelle :

L, représente un hétéroatome de préférence choisi parmi l'oxygène, le soufre ; un groupe NH ; un groupe N-$R_{34}$,

M représente un hétéroatome de préférence choisi parmi l'oxygène, le soufre, l'azote ; un groupe $N^{+}-R_{34}$ ; un groupe CH ; un groupe $C-R_{35}$ ;

Q, K, identiques ou non, représentent un groupement hydroxyle ; un radical amino ; un radical amino substitué par un ou plusieurs radicaux alkyle, identiques ou non, linéaires ou ramifiés, en $C_1$-$C_8$, éventuellement porteurs d'au moins un groupement hydroxyle, un radical aryle éventuellement substitué, un radical alkyl($C_1$-$C_8$)aryle éventuellement substitué ; un groupement ammonium de type $N+(R_{36})_t$ avec t égal à 2 pour K et à 3 pour Q,

$R_{36}$, identiques ou non, représentent un atome d'hydrogène ; un radical alkyle, linéaire ou ramifié, en $C_1$-$C_8$, éventuellement porteur d'au moins un groupement hydroxyle ; un radical aryle éventuellement substitué, un radical alkyl($C_1$-$C_8$)aryle la partie aryle étant éventuellement substituée ; un radical alkyle en C1-C8, linéaire ou ramifiée, éventuellement substituée, un radical alcoxy, linéaire ou ramifié, en $C_1$-$C_8$, éventuellement substitué ;

A la condition que Q et K ne représentent pas à la fois un groupement ammonium de type $N^{+}(R_{36})_t$ ;

$R_{32}$, $R_{33}$, identiques ou non, représentent un atome d'hydrogène ; un radical alkyle, linéaire ou ramifié, en $C_1$-$C_8$ éventuellement substitué ; un radical amino ; un radical amino substitué ou non par un ou plusieurs radicaux, identiques ou non, choisis parmi les radicaux alkyle, linéaires ou ramifiés, en $C_1$-$C_8$ éventuellement substitués, les radicaux phényle éventuellement substitués ; un atome d'halogène de préférence le chlore, le fluor ;

Au cas où Q représente un radical amino substitué ou non, ou un groupement hydroxyle, $R_{32}$ peut représenter un radical alkylamino ou alcoxy formant avec l'atome d'azote ou d'oxygène du radical **J**, un cycle à 6 chaînons, éventuellement condensé avec un radical aromatique, ledit radical aromatique étant éventuellement substitué par au moins un groupement amino ou amino substitué ou non par un ou plusieurs radicaux identiques ou non, choisis parmi les radicaux alkyle, linéaires ou ramifiés, en $C_1$-$C_8$ éventuellement substitués, les radicaux phényle éventuellement substitués ;

$R_{34}$, $R_{35}$, identiques ou non, représentent un radical alkyle, linéaire ou ramifié en $C_1$-$C_8$ éventuellement substitué, de préférence par au moins un hydroxyle ; au moins un radical alcoxy, linéaire ou ramifié en $C_1$-$C_8$ ; au moins un radical amino ; au moins un radical amino substitué par un ou plusieurs radicaux alkyles, identiques ou non, linéaires ou ramifiés, en $C_1$-$C_8$, éventuellement porteurs d'au moins un radical hydroxyle.

$R_{34}$, $R_{35}$, identiques ou non, peuvent aussi représenter un radical aryle, de préférence en $C_6$, éventuellement substitué, de préférence par au moins un radical alkyle, linéaire ou ramifié, en $C_1$-$C_8$ ; par au moins un groupement hydroxyle ; par au moins un radical alcoxy, linéaire ou ramifié, en $C_1$-$C_8$ ; par au moins un radical amino ; par au moins un radical amino substitué par un ou plusieurs radicaux alkyle, identiques ou non, linéaires ou ramifiés, en $C_1$-$C_8$, éventuellement porteurs d'au moins un groupement hydroxyle ; un atome d'halogène et de préférence le chlore ou le fluor ; par au moins un groupement nitro ; par au moins un groupement cyano.

**19.** Composition selon la revendication 10, **caractérisée en ce que** le chromophore de la famille des composés nitrés (hétéro)aromatiques correspondant aux formules (XIII) et (XIV) ci-dessous, ainsi que leurs formes tautomères :

| | |
|---|---|
| benzénique | 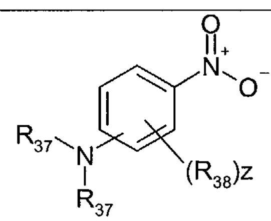 (XIII) |
| Pyridinique | 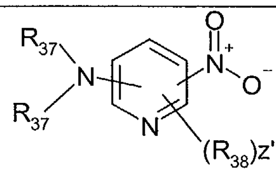 (XIV) |

Formules dans lesquelles

$R_{37}$, identiques ou non, représentent un atome d'hydrogène ; un radical alkyle, linéaire ou ramifié, en $C_1$-$C_8$, éventuellement porteur d'au moins un groupement hydroxyle ; un radical aryle éventuellement substitué, un radical alkyl($C_1$-$C_8$)aryle la partie aryle étant éventuellement substituée ; un radical alkyle en C1-C8, linéaire ou ramifiée, éventuellement substituée, un radical alcoxy, linéaire ou ramifié, en $C_1$-$C_8$, éventuellement

substitué ;
$R_{38}$, identiques ou différents, représentent un atome d'hydrogène ; alkyle linéaire ou ramifié en $C_1$-$C_4$, éventuellement substitué ; un radical alcoxy linéaire ou ramifié en $C_1$-$C_8$, éventuellement substitué ; un radical aryle en $C_6$ éventuellement substitué ; un radical amino ; un radical amino substitué par un ou plusieurs radicaux choisis parmi les radicaux alkyle, identiques ou non, linéaires ou ramifiés, en $C_1$-$C_8$, de préférence en $C_1$-$C_4$, éventuellement substitués, de préférence par au moins un groupe hydroxyle, alcoxy, linéaire ou ramifié en $C_1$-$C_4$, thioalkyl linéaires ou ramifiés en $C_1$-$C_4$, alkylsulfonamido linéaires ou ramifiés en $C_1$-$C_4$, parmi les radicaux aryle en $C_6$ éventuellement substitué et parmi les radicaux hétéroaryle comprenant 5 à 6 chaînons éventuellement substitués ; un groupe hydroxyle ; un groupe nitro ; un groupe cyano ;
Le coefficient z étant égal à 4 et le coefficient z' étant égal à 3.

**20.** Composition selon l'une quelconque des revendications précédentes, **caractérisée en ce que** le bras de liaison est cationique ou non cationique.

**21.** Composition selon l'une quelconque des revendications précédentes, **caractérisée en ce que** le bras de liaison est choisi parmi les composés divalents, trivalents ou tétravalents et de préférence parmi une chaîne hydrocarbonée en $C_1$-$C_{20}$, de préférence en $C_1$-$C_{12}$, linéaire ou ramifiée, de préférence une chaîne alkyle, un ou plusieurs des atomes de carbone de la chaîne pouvant être remplacés par un hétéroatome tel que le soufre, l'azote, l'oxygène, par un hétérocycle en $C_5$-$C_6$, saturé ou non, comprenant de préférence au moins deux atomes d'azote ; la chaîne hydrocarbonée pouvant être insaturée ou contenir un radical arylène ; un radical arylène ; un radical divalent téréphtalamide ; un radical divalent ou trivalent, par exemple de type triazine.

**22.** Composition selon l'une quelconque des revendications précédentes, **caractérisée en ce que** la teneur en colorant mixte varie de 0,001 à 20% en poids, plus particulièrement, de 0,005 à 10% en poids, et de préférence, de 0,01 et 5% en poids, par rapport au poids total de la composition.

**23.** Composition selon l'une quelconque des revendications précédentes, **caractérisée en ce qu'**elle comprend un ou plusieurs colorants directs additionnels autres que ledit ou lesdits colorants mixtes.

**24.** Composition selon la revendication précédente, **caractérisée en ce que** la teneur en chacun des colorants directs additionnels est varie de 0,001 à 10 % en poids par rapport au poids total de la composition tinctoriale.

**25.** Composition selon l'une quelconque des revendications précédentes, **caractérisée en ce qu'**elle comprend au moins une base d'oxydation choisie parmi les paraphénylènediamines, les bis-phénylalkylènediamines, les para-aminophénols, les ortho-aminophénols, les bases hétérocycliques et leurs sels d'addition.

**26.** Composition selon l'une quelconque des revendications précédentes, **caractérisée en ce qu'**elle comprend au moins un coupleur choisi parmi les métaphénylènediamines, les méta-aminophénols, les métadiphénols, les coupleurs naphtaléniques, les coupleurs hétérocycliques et leurs sels d'addition.

**27.** Composition selon l'une quelconque des revendications 25 ou 26, **caractérisée en ce que** la teneur de chacune des bases d'oxydation est varie de 0,001 à 10 % en poids par rapport au poids total de la composition tinctoriale et la teneur de chacun des coupleurs varie de 0,001 à 10 % en poids par rapport au poids total de la composition tinctoriale.

**28.** Composition selon l'une quelconque des revendications précédentes, **caractérisée en ce que** la composition comprend au moins un agent oxydant.

**29.** Composition selon la revendication précédente, **caractérisée en ce que** l'agent oxydant est choisi parmi le peroxyde d'hydrogène, les peroxydes de métaux alcalins ou alcalino-terreux, le peroxyde d'urée, les bromates ou ferricyanures de métaux alcalins, les persels, les enzymes telles que les peroxydases et les oxydo-réductases à deux ou à quatre électrons, et de préférence le peroxyde d'hydrogène.

**30.** Composition selon l'une quelconque des revendications précédentes, **caractérisée en ce que** le pH est compris entre 8 et 11.

**31.** Procédé de coloration de fibres kératiniques, notamment humaines, consistant à mettre en oeuvre les étapes suivantes :

a) on applique la composition selon l'une quelconque des revendications 1 à 27, sur de fibres sèches ou non, éventuellement en présence d'au moins un agent oxydant,
b) on laisse poser pendant une durée suffisante pour obtenir la coloration souhaitée,
c) on rince éventuellement les fibres,
d) on lave les fibres et on les rince,
e) on sèche ou on laisse sécher les fibres.

**32.** Dispositif à plusieurs compartiments dans lequel au moins un premier compartiment contient une composition tinctoriale comprenant au moins un colorant mixte tel que décrit dans l'une des revendications 1 à 21, et éventuellement au moins un colorant direct additionnels différent du ou des colorants mixtes, éventuellement au moins une base d'oxydation, éventuellement au moins un coupleur, et un autre compartiment contenant un agent oxydant.

**33.** Colorant mixte tel que défini à l'une quelconque des revendications 1 à 21, à l'exception des composés suivants :

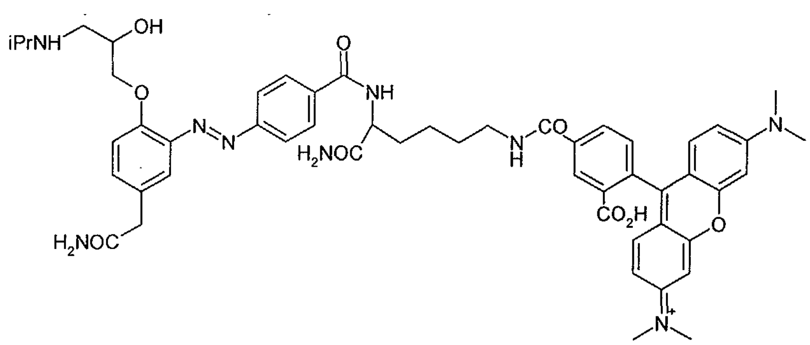

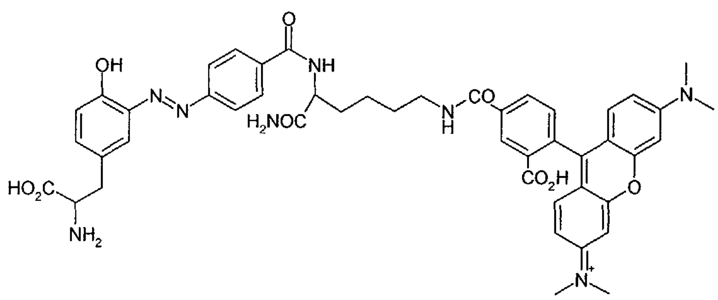

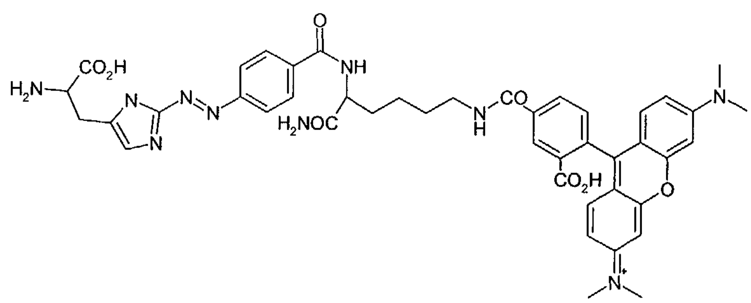

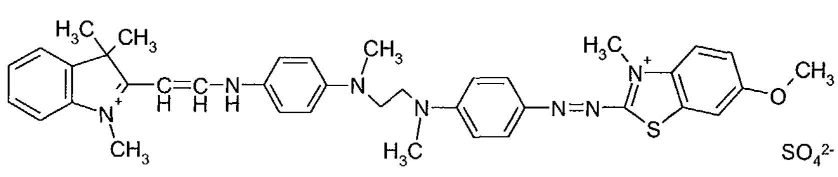

**34.** Colorant mixte selon la revendication précédente, correspondant à l'une des formules suivantes, et leurs sels d'addition :